# EUROPEAN PATENT SPECIFICATION

(11) **EP 3 959 326 B1**
(45) Date of publication and mention of the grant of the patent: **07.06.2023**
(21) Application number: 20734422.7
(22) Date of filing: 30.06.2020
(51) Int. Cl.: C12P 1/00, C12P 21/00, C12P 21/02

(54) **INDUSTRIAL FERMENTATION PROCESS FOR MICROBIAL CELLS USING A FED-BATCH PRE-CULTURE**
INDUSTRIELLES FERMENTATIONSVERFAHREN FÜR MIKROBIELLE ZELLEN UNTER VERWENDUNG EINER FED-BATCH-VORKULTUR
PROCÉDÉ DE FERMENTATION INDUSTRIEL POUR CELLULES MICROBIENNES À L'AIDE D'UNE PRÉCULTURE D'ÉCOULEMENT CONTINU ENZYMATIQUE

(30) Priority: 05.07.2019 EP 19184580; 05.05.2020 EP 20170663
(43) Date of publication of application: 02.03.2022
(73) Proprietor: BASF SE, 67056 Ludwigshafen am Rhein (DE)
(72) Inventor: KLEIN, Tobias, 67056 Ludwigshafen (DE); DAUB, Andreas, 67056 Ludwigshafen (DE); GOLABGIR ANBARANI, Aydin, 67056 Ludwigshafen (DE)
(74) Representative: BASF IP Association
(86) International application number: PCT/EP2020/068335
(87) International publication number: WO 2021/004830

(56) References cited:
- WO-A1-2017/097869
- JP-A- S6 128 381
- R. HUBER ET AL: "Equalizing growth in high-throughput small scale cultivations via precultures operated in fed-batch mode", BIOTECHNOLOGY AND BIOENGINEERING, vol. 103, no. 6, 15 August 2009 (2009-08-15), pages 1095-1102, XP055651979, ISSN: 0006-3592, DOI: 10.1002/bit.22349
- J. OTTEN ET AL: "ACCELERATED YOGHOURT PRODUCTION BY FED-BATCH PREFERMENTATION", NETHERLANDS MILK AND DAIRY JOURNAL, PUDOC. WAGENINGEN, NL, vol. 50, no. 1, 1996, pages 19-34, XP000580301, ISSN: 0028-209X
- U. E. GIESECKE ET AL: "Production of alkaline protease with Bacillus lichenifortnis in a controlled fed-batch process", APPL MICROBIOL BIOTECHNOL, vol. 35, 1991, pages 720-724, XP055610412,
- CLAUDIA KORNELI ET AL: "Influence of fructose and oxygen gradients on fed-batch recombinant protein production using Bacillus megaterium", ENGINEERING IN LIFE SCIENCES, vol. 11, no. 4, August 2011 (2011-08), pages 338-349, XP055726365, DE ISSN: 1618-0240, DOI: 10.1002/elsc.201000161
- MARIAN WENZEL ET AL: "Self-Inducible Bacillus subtilis Expression System for Reliable and Inexpensive Protein Production by High-Cell-Density Fermentation", APPLIED AND ENVIRONMENTAL MICROBIOLOGY, vol. 77, no. 18, 15 September 2011 (2011-09-15), pages 6419-6425, XP055616282, US ISSN: 0099-2240, DOI: 10.1128/AEM.05219-11

## Description

### Field of the invention

The invention relates to a process for culturing Bacillus cells producing a protein of interest comprising a fed-batch pre-culture.

### Background

Microorganisms are widely used as industrial workhorses for the production of a product of interest, especially proteins, and in particular enzymes. The biotechnological production of the product of interest is conducted via fermentation and subsequent purification of the product. Microorganisms, like the *Bacillus species,* are capable of secreting significant amounts of product into the fermentation broth. This allows a simple product purification process compared to intracellular production and explains the success of *Bacillus* in industrial application.

WO-A- 2017/097869 discloses a method for culturing Bacillus cells, preferably of Bacillus subtilis, Bacillus pumilus, Bacillus licheniformis, or Bacillus lentus, for producing a protein and a process for producing a protein by culturing Bacillus cells. The protein produced is an enzyme such as an amylase, protease, lipase, mannanase, phytase, and cellulase, preferably, amylase or protease.

R. HUBER ET AL: "Equalizing growth in high-throughput small scale cultivations via precultures operated in fedbatch mode", BIOTECHNOLOGY AND BIOENGINEERING, vol. 103, no. 6, 15 August 2009, pages 1095-1102, teaches a process for culturing microbial cells from E. coli and Hansenula polymorpha to produce glucose comprising the provision of a pre-culture and culturing of the pre-culture in a fed-batch mode.

JP S61 28381 A discloses a process for culturing as Streptococcus thermophilus (FERM P-7025) cells to produce glubatch modecose wherein a pre-culture is performed in a fed-batch mode.

J. OTTEN ET AL: "Accelerated Yoghourt Production by Fed-batch Prefermentation", NETHERLANDS MILK DAIRY JOURNAL, vol. 50, no. 1, 1996, pages 19-34, teaches a process for culturing Streptococcus and Lactobacillus delbrueckiil cells and the production of yogurt comprising a pre-culture and culturing in a fedbatch mode followed by batch culturing to produce yogurt.

Industrial bioprocesses using microorganisms are typically performed in large-scale production bioreactors having a size of more than 50 m³. A pre-culture consisting of at least one cultivation step in smaller seed fermenters is used to produce the required amount of inoculum for the production in these large-scale production bioreactors. A pre-culture consisting of more than one cultivation step, i.e. involving the subsequent use of more than one seed fermenter, is most often referred to as a seed train. The seed fermenters are usually run in batch mode. The volume of the inoculum for inoculation of the main bioreactor can range from 0.1 to 15% (v/v) of the volume of the initial volume of the production bioreactor. Depending on the amount of inoculum, the duration of the main fermentation can vary. A larger inoculation volume contains more biomass and can therefore significantly decrease the time until harvest of the desired product. However, an inoculation volume larger than 15% (v/v) of the initial volume of the production bioreactor is not reasonable from a technical and economical point of view. Thus, a new process for culturing of microbial cells is needed, which is able to reduce the time until harvest of the product of interest without exceeding the inoculation volume above 15% of the initial volume of the production bioreactor.

### Summary of the invention

The inventors found out that a pre-culture run in fed-batch mode can be used to increase the cell density in the pre-culture seed fermenter, thus increasing the amount of biomass contained in the inoculum. The herein described inoculum enriched with cells can be used to shorten the time until harvest of the fermenter compared to a normal pre-culture, which is run in batch mode, without increasing the volume of inoculum introduced into the production bioreactor.

Therefore, in a first aspect the present invention relates to a process for culturing Bacillus cells producing a protein of interest, comprising the steps of:
(a) providing a pre-culture of said microbial cells;
(b) inoculating a production bioreactor with the pre-culture of step (a); and
(c) culturing the microbial cells in the production bioreactor under conditions conducive for the production of the protein of interest,
wherein the pre-culture of step (a) is performed in fed-batch mode, and
wherein the Bacillus cells are cells of Bacillus subtilis, Bacillus pumilus, Bacillus licheniformis, or Bacillus lentus.

In one embodiment, the volume of the pre-culture used for inoculating the production bioreactor is not more than 15% of the volume of the fermentation medium present in the production bioreactor.

In another embodiment, the pre-culture comprises at least two stages, wherein at least the last stage of the at least two stages is performed in fed-batch mode.

In another embodiment, culturing the Bacillus cells in the production bioreactor is performed in batch mode, fed-batch mode or continuous fermentation mode.

In a preferred embodiment, the protein is an enzyme.

Preferably, the protein of interest is not a reporter protein, preferably not GFP or YFP.

The enzyme may be selected from the list consisting of hydrolases, oxidases, isomerases, e.g. amylase, alpha-amylase, glucoamylase, pullulanase, protease, metalloprotease, peptidase, lipase, cutinase, acyl transferase, cellulase, endoglucanase, glucosidase, cellubiohydrolase, xylanase, xyloglucantransferase, xylosidase, mannanase, phytase, phosphatase, xylose isomerase, glucose isomerase, lactase, acetolactate decarboxylase, pectinase, pectin methylesterase, polygalacturonidase, lyase, pectate lyase, arabinase, arabinofuranosidase, galactanase, laccase, peroxidase and asparaginase.

In one embodiment, the protein of interest is secreted by the Bacillus cells into the fermentation broth.

In another embodiment, the process of the present invention further comprises a step (d) of harvesting the product when the concentration of the protein of interest is at least 10g protein/kg fermentation broth.

In another aspect, the invention relates to a process for producing a protein of interest, comprising the steps of:
(a) providing a pre-culture of Bacillus cells, producing the protein of interest;
(b) inoculating a production bioreactor with the pre-culture of step (a); and
(c) culturing the Bacillus cells in the production bioreactor under conditions conducive for the production of the protein of interest,
wherein the pre-culture of step (a) is performed in fed-batch mode, and
wherein the Bacillus cells are cells of Bacillus subtilis, Bacillus pumilus, Bacillus licheniformis, or Bacillus lentus.

In a further aspect, the invention relates to a process for increasing the yield of a protein of interest, comprising the steps of:
(a) providing a pre-culture of Bacillus cells, producing the protein of interest;
(b) inoculating a production bioreactor with the pre-culture of step (a); and
(c) culturing the Bacillus cells in the production bioreactor under conditions conducive for the production of the protein of interest,
wherein the pre-culture of step (a) is performed in fed-batch mode, and
wherein the Bacillus cells are cells of Bacillus subtilis, Bacillus pumilus, Bacillus licheniformis, or Bacillus lentus.

In a further aspect, the invention relates to process for reducing the time until harvest in the fermentative production a compound of interest, in particular a protein of interest, comprising the steps of:
(a) providing a pre-culture of Bacillus cells, producing said compound of interest;
(b) inoculating a production bioreactor with the pre-culture of step (a); and
(c) culturing the Bacillus cells in the production bioreactor under conditions conducive for the production of the compound of interest,
wherein the pre-culture of step (a) is performed in fed-batch mode, and
wherein the Bacillus cells are cells of Bacillus subtilis, Bacillus pumilus, Bacillus licheniformis, or Bacillus lentus.

### Brief description of the drawings

Figure 1 illustrates the relative time until harvest in a production process using either a batch or a fed-batch pre-culture for the production of (A) a protease and (B) an amylase. In each case, results were normalized to the time until harvest of the pre-culture run in batch mode, which is set to 100.
Figure 2 illustrates the relative time until harvest in a production process using either a batch or a fed-batch pre-culture and inoculating the production bioreactor with an inoculum having a volume of either 10% or 1% (v/v) of the initial volume of the fermentation medium present in the production bioreactor. For each volume, results were normalized to the time until harvest of the pre-culture run in batch mode.
Figure 3 illustrates the relative time until harvest in a production process using either a chemically defined medium or complex medium in the seed fermenter of the pre-culture. Results were normalized to the time until harvest of the pre-culture conducted with chemically defined medium.
Figure 4 illustrates the relative time until harvest in a production process using either a batch or a fed-batch pre-culture for the production of a protease in Bacillus subtilis.

### Definitions

Unless otherwise noted, the terms used herein are to be understood according to conventional usage by those of ordinary skill in the relevant art.

As used in this specification and in the appended claims, the singular forms of "a" and "an" also include the respective plurals unless the context clearly dictates otherwise. In the context of the present invention, the terms "about" and "approximately" denote an interval of accuracy that a person skilled in the art will understand to still ensure the technical effect of the feature in question. The term typically indicates a deviation from the indicated numerical value of ±20 %, preferably ±15 %, more preferably ±10 %, and even more preferably ±5 %.

It is to be understood that the term "comprising" is not limiting. For the purposes of the present invention the term "consisting of" is considered to be a preferred embodiment of the term "comprising". If hereinafter a group is defined to comprise at least a certain number of embodiments, this is meant to also encompass a group, which preferably consists of these embodiments only.

Furthermore, the terms "first", "second", "third" or "(a)", "(b)", "(c)", "(d)" etc. and the like in the description and in the claims are used for distinguishing between similar elements and not necessarily for describing a sequential or chronological order. It is to be understood that the terms so used are interchangeable under appropriate circumstances and that the embodiments of the invention described herein are capable of operation in other sequences than described or illustrated herein. In case the terms "first", "second", "third" or "(a)", "(b)", "(c)", "(d)", "i", "ii" etc. relate to steps of a method or use or assay, there is no time or time interval coherence between the steps, i.e. the steps may be carried out simultaneously or there may be time intervals of seconds, minutes, hours, days, weeks, months or even years between such steps, unless otherwise indicated in the application as set forth herein above or below.

It is to be understood that this invention is not limited to the particular methodology, protocols, reagents etc. described herein as these may vary. It is also to be understood that the terminology used herein is for the purpose of describing particular embodiments only, and is not intended to limit the scope of the present invention that will be limited only by the appended claims. Unless defined otherwise, all technical and scientific terms used herein have the same meanings as commonly understood by one of ordinary skill in the art.

Throughout this application, various publications are referenced.

A "process for culturing Bacillus cells producing a protein of interest" or "fermentation process" comprises the cultivation of Bacillus cells in a suitable fermentation medium and under suitable conditions such as a suitable temperature and suitable pH. "Cultivation of the cells" or "growth of the cells" is not understood to be limited to an exponential growth phase, but can also include the physiological state of the cells at the beginning of growth after inoculation and during a stationary phase.

The term "fermentation broth" or "culture broth" as used herein describes the fermentation medium containing Bacillus cells, which are cultivated to express and, depending on the protein of interest, secrete the protein of interest into the fermentation medium.

The term "fermentation medium" refers to a water-based solution containing one or more chemical compounds that can support the growth of cells.

The term "feed solution" is used herein for a solution that is added during the fermentation process to the fermentation medium after inoculation of the initial fermentation medium with the Bacillus cells. The feed solution comprises compounds supportive for the growth of the cells. In one embodiment, the feed solution has the same composition as the initial fermentation medium. In another embodiment, the feed solution has a composition which is different from the composition of the fermentation medium. Compared to the fermentation medium the feed solution may be enriched for one or more compounds.

The term "pre-culture" refers to a liquid actively growing culture of the Bacillus cell which is cultivated in a seed fermenter and which is used for inoculating the "production bioreactor". "Actively growing" is intended to mean that the culture is in a stage where the number of Bacillus cells in the culture increases. Thus, at the beginning of the pre-culture the cells may be in a lag phase and switch to an exponential growth phase over time. The cells of the pre-culture are in general used as inoculation material in order to avoid or reduce the lag phase in the production bioreactor. Thus, at the time point of the transfer of the pre-culture cells into the production bioreactor, or into the next seed fermenter of a seed train, the cells are preferably in exponential phase or in late exponential phase where the cells are growing actively.

The term "seed fermenter" refers to a culture vessel in which the pre-culture is formed by fermenting Bacillus cells until a sufficiently high number of cells for inoculation into the main fermenter or into the next seed fermenter of a seed train is obtained. The seed fermenter has a smaller volume than the production bioreactor. In one embodiment, the volume of the seed fermenter is between 5% and 20% of the volume of the production reactor. In one embodiment, the volume of the seed fermenter is between 8% and 15% of the volume of the production reactor. In one embodiment, the volume of the seed fermenter is 10% of the volume of the production reactor.

The term "seed train" is intended to mean a series of seed fermenters of increasing size in which the pre-culture is conducted in a series of fermenters where the last fermenter in the seed train has a sufficient size to contain the necessary inoculum for the production bioreactor. Hence, the volume of the last seed fermenter in the seed train is between 5% and 20% of the volume of the production reactor. In one embodiment, the volume of the last seed fermenter in the seed train is between 8% and 15% of the volume of the production reactor. In one embodiment, the volume of the last seed fermenter in the seed train is 10% of the volume of the production reactor. The volume of the seed fermenters within a seed train may increase 5 to 20 fold from one seed fermenter to the next seed fermenter, until a sufficient amount of cells to inoculate the production bioreactor has been obtained.

The term "production bioreactor" or "main bioreactor" or "main fermenter" or "production fermenter" is a term known in the art and is intended to mean a vessel in which the cultivation of cells in large scale and the large-scale production of a protein of interest takes place. "Large scale production", "Large scale fermentation" or also called herein "industrially relevant fermentation" refers to a fermentation process in which more than 200 g of a carbon source (preferably, a chemically defined carbon source) per liter of initial fermentation medium are added to the production bioreactor during culturing of the cells in the production bioreactor. Hence, culturing cells in a "production bioreactor" or "main bioreactor" or "main fermenter" or "production fermenter" refers to a fermentation process in which more than 200 g of a carbon source (preferably, a chemically defined carbon source) per liter of initial fermentation medium are added to the production bioreactor during culturing of the cells in the production bioreactor. After termination of the cultivation in the production bioreactor the fermentation broth is harvested and the protein of interest is recovered. The bioreactor may contain inlets and outlets, for example for media, and different sensors, e.g. for measuring pH and temperature during the fermentation process. The fermentation medium in the production bioreactor may be the same as or different from the fermentation medium used in the seed fermenter or the last seed fermenter in a seed train.

The production bioreactor may have a volume of at least 500 L, at least 1,000 L, at least 5,000 L, at least 10,000 L, at least 20,000 L, at least 50,000L, or at least 100,000 L. Preferably, the production bioreactor may have a volume of 500-1000 L, 1,000-5,000 L, at 5,000-10,000 L, 10,000-20,000 L, 20,000-50,000 L, 50,000-100,000 L, or 100,000-150,000 L. The production bioreactor may have a volume of 500 L, 1,000 L, 5,000 L, 10,000 L, 20,000 L 50,000L, or 100,000 L.

If the production bioreactor has a volume of 500 L, the volume of the seed fermenter may be between 25 L and 100 L, or between 40 L and 75 L or 50 L. If the production bioreactor has a volume of 1,000 L, the volume of the seed fermenter may be between 50 L and 200 L, or between 80 L and 150 L or 100 L. If the production bioreactor has a volume of 5,000 L, the volume of the seed fermenter may be between 250 L and 1,000 L, or between 400 L and 750 L or 500 L. If the production bioreactor has a volume of 10,000 L, the volume of the seed fermenter may be between 500 L and 2,000 L, or between 800 L and 1,500 L or 1,000 L. If the production bioreactor has a volume of 20,000 L, the volume of the seed fermenter may be between 1,000 Land 4,000 L, or between 1,600 Land 3,000 L or 2,000 L. If the production bioreactor has a volume of 50,000 L, the volume of the seed fermenter may be between 2,500 L and 10,000 L, or between 4,000 Land 7,500 L or 5,000 L. If the production bioreactor has a volume of 10,000 L, the volume of the seed fermenter may be between 5,000 L and 20,000 L, or between 8,000 Land 15,000 L or 10,000 L.

If the production bioreactor has a volume of 1,000 L, the volume of the last seed fermenter in the seed train may be between 50 L and 200 L, or between 80 L and 150 L or 100 L. If the production bioreactor has a volume of 5,000 L, the volume of the last seed fermenter in the seed train may be between 250 L and 1,000 L, or between 400 L and 750 L or 500 L. If the production bioreactor has a volume of 10,000 L, the volume of the last seed fermenter in the seed train may be between 500 L and 2,000 L, or between 800 L and 1,500 L or 1,000 L. If the production bioreactor has a volume of 20,000 L, the volume of the last seed fermenter in the seed train may be between 1,000 Land 4,000 L, or between 1,600 L and 3,000 L or 2,000 L. If the production bioreactor has a volume of 50,000 L, the volume of the last seed fermenter in the seed train may be between 2,500 Land 10,000 L, or between 4,000 Land 7,500 L or 5,000 L. If the production bioreactor has a volume of 100,000 L, the volume of the last seed fermenter in the seed train may be between 5,000 Land 20,000 L, or between 8,000 Land 15,000 L or 10,000 L.

The term "inoculum" is intended to mean an amount of the Bacillus cells that is added from the seed fermenter to the production bioreactor in order to start the fermentation process in the production bioreactor. Furthermore, "inoculum" is also intended to mean an amount of the Bacillus cells from a seed fermenter that is added to the subsequent seed fermenter in a seed train.

The term "batch mode" or "batch fermentation" refers to a culture mode wherein the cells are cultured in the initially present fermentation medium without any change in medium composition or the volume of the medium. Thus, in batch mode no substantial or significant amount of fresh liquid culture medium is added to the cell culture and no substantial or significant amount of liquid culture medium is removed from the cell culture during culturing.

The term "fed-batch mode" or "fed-batch fermentation" refers to a culture mode wherein the cells are cultured in the initially present fermentation medium and a feed solution is added in a periodic or continuous manner without substantial or significant removal of liquid culture medium during culturing. Fed-batch cultures can include various feeding regimens and times, for example, daily feeding, feeding more than once per day, or feeding less than once per day, and so on.

The term "continuous fermentation mode" or "continuous fermentation" refers to a culture mode wherein the cells are cultured in the initially present fermentation medium and new fermentation medium is continuously fed to the fermenter and ferment is removed from the fermenter at the same rate so that the volume in the fermenter is constant.

The term "titer of a protein of interest" as used herein is understood as the amount of protein of interest in g per volume of fermentation broth in liter (g/L).

The term "harvesting" (as in "harvesting the protein of interest") refers to separation of the protein of interest from at least a part of the biomass in the fermentation medium. Harvesting can be done by any known method, such as filtration and centrifugation. The harvesting method is preferably adjusted to the needs and characteristics of the specific protein of interest.

The term "time until harvest" or "time to harvest" as used herein is defined as the period between the inoculation of the production bioreactor and the time point at which the titer of a protein of interest reaches a certain amount measured in g protein / kg fermentation broth. The time until harvest may be the period between the inoculation of the production bioreactor and the time point at which the titer of a protein of interest reaches 2 to 20 g protein / kg fermentation broth. In one embodiment, the time until harvest is the period between the inoculation of the production bioreactor and the time point at which the titer of a protein of interest reaches 5 to 15 g protein / kg fermentation broth. In one embodiment, the time until harvest may be the period between the inoculation of the production bioreactor and the time point at which the titer of a protein of interest reaches 8 to 12 g protein / kg fermentation broth. In one embodiment, the time until harvest may be the period between the inoculation of the production bioreactor and the time point at which the titer of a protein of interest reaches 10 g protein / kg fermentation broth. The time until harvest may be the period between the inoculation of the production bioreactor and the time point at which the titer of a protein of interest reaches at least 2 g protein / kg fermentation broth, at least 5 g protein / kg fermentation broth, at least 10 g protein / kg fermentation broth, at least 15 g protein / kg fermentation broth, or at least 20 g protein / kg fermentation broth, preferably at least 10 g protein / kg fermentation broth. In one embodiment, the time until harvest is the period between the inoculation of the production bioreactor and the time point at which the titer of a protein of interest reaches 5 to 15 g protein / kg fermentation broth. In one embodiment, the time until harvest may be the period between the inoculation of the production bioreactor and the time point at which the titer of a protein of interest reaches 8 to 12 g protein / kg fermentation broth. In one embodiment, the time until harvest may be the period between the inoculation of the production bioreactor and the time point at which the titer of a protein of interest reaches 10 g protein / kg fermentation broth. The time until harvest may be the period between the inoculation of the production bioreactor and the time point at which the titer of a protein of interest reaches at least 2 g protein / kg fermentation broth, at least 5 g protein / kg fermentation broth, at least 10 g protein / kg fermentation broth, at least 15 g protein / kg fermentation broth, or at least 20 g protein / kg fermentation broth, preferably at least 10 g protein / kg fermentation broth. The time until harvest may be the period between the inoculation of the production bioreactor and the time point at which the titer of a protein of interest reaches 2 g protein / kg fermentation broth, 5 g protein / kg fermentation broth, 10 g protein / kg fermentation broth, 15 g protein / kg fermentation broth, or 20 g protein / kg fermentation broth, preferably 10 g protein / kg fermentation broth.

The term "heterologous" (or exogenous or foreign or recombinant or non-native) polypeptide is defined herein as a polypeptide that is not native to the host cell, a polypeptide native to the host cell in which structural modifications, e.g., deletions, substitutions, and/or insertions, have been made by recombinant DNA techniques to alter the native polypeptide, or a polypeptide native to the host cell whose expression is quantitatively altered or whose expression is directed from a genomic location different from the native host cell as a result of manipulation of the DNA of the host cell by recombinant DNA techniques, or whose expression is quantitatively altered as a result of manipulation of the regulatory elements of the polynucleotide by recombinant DNA techniques, e.g. by using a stronger promoter; or a polynucleotide native to the host cell, but integrated not within its natural genetic environment as a result of genetic manipulation by recombinant DNA techniques.

With respect to two or more polynucleotide sequences or two or more amino acid sequences, the term "heterologous" is used to characterize that the two or more polynucleotide sequences or two or more amino acid sequences are naturally not occurring in the specific combination with each other.

For the purposes of the invention, "recombinant" (or transgenic) with regard to a cell or an organism means that the cell or organism contains a heterologous polynucleotide which is introduced by man by gene technology and with regard to a polynucleotide the term "recombinant" includes all those constructions brought about by man by gene technology / recombinant DNA techniques in which either
(a) the sequence of the polynucleotide or a part thereof, or
(b) one or more genetic control sequences which are operably linked with the polynucleotide, including, but not limited thereto, a promoter, or
(c) both a) and b) are not located in their wildtype genetic environment or have been modified.

The term "native" (or wildtype or endogenous) cell or organism and "native" (or wildtype or endogenous) polynucleotide or polypeptide refers to the cell or organism as found in nature and to the polynucleotide or polypeptide in question as found in a cell in its natural form and genetic environment, respectively (i.e., without there being any human intervention).

### Detailed description

The present invention may be understood more readily by reference to the following detailed description of the embodiments of the invention and the examples included herein.

The present invention is directed to an industrially relevant fermentation process for producing a protein of interest in Bacillus cells. The inventors surprisingly found that a pre-culture step run in fed-batch mode can be used to increase the cell density in the seed fermenter, thereby increasing the amount of biomass contained in the inoculum for inoculation of the production bioreactor without increasing the volume of the inoculum. The enriched inoculum can be used to shorten the time until harvest of the protein of interest compared to a fermentation process wherein the pre-culture is run in batch mode.

Therefore, in a first aspect the invention relates to a process for culturing Bacillus cells producing a protein of interest, comprising the steps of:
(a) providing a pre-culture of said Bacillus cells;
(b) inoculating a production bioreactor with the pre-culture of step (a); and
(c) culturing the Bacillus cells in the production bioreactor under conditions conducive for the production of the protein of interest,
wherein the pre-culture of step (a) is performed in fed-batch mode, and
wherein the Bacillus cells are cells of Bacillus subtilis, Bacillus pumilus, Bacillus licheniformis, or Bacillus lentus.

In a second aspect, the invention relates to a process for producing a protein of interest, comprising the steps of:
(a) providing a pre-culture of Bacillus cells producing said protein of interest;
(b) inoculating a production bioreactor with the pre-culture of step (a); and
(c) culturing the Bacillus cells in the production bioreactor under conditions conducive for the production of the protein of interest,
wherein the pre-culture of step (a) is performed in fed-batch mode, and
wherein the Bacillus cells are cells of Bacillus subtilis, Bacillus pumilus, Bacillus licheniformis, or Bacillus lentus.

In a further aspect, the invention relates to a process for increasing the yield of a product of interest, comprising the steps of:
(a) providing a pre-culture of Bacillus cells producing said protein of interest;
(b) inoculating a production bioreactor with the pre-culture of step (a); and
(c) culturing the Bacillus cells in the production bioreactor under conditions conducive for the production of the protein of interest,
wherein the pre-culture of step (a) is performed in fed-batch mode, and
wherein the Bacillus cells are cells of Bacillus subtilis, Bacillus pumilus, Bacillus licheniformis, or Bacillus lentus.

### Step a): the pre-culture

The majority of the protein of interest is produced by culturing the Bacillus cells in the production bioreactor. Therefore, the fermentation medium present in the production bioreactor has to be inoculated with a starting culture of these Bacillus cells, the inoculum.

In general, the inoculum is a liquid culture of the Bacillus cells, which is prepared in a seed fermenter.

In one embodiment, the pre-culture of the Bacillus cells uses only one seed fermenter, i.e. only one pre-culture step.

In another embodiment, the pre-culture of Bacillus cells uses more than one pre-culture step conducted using more than one seed fermenter. A pre-culture involving more than one seed fermenter is often referred to as seed train. A seed train for an industrial-scale production process may comprise one to four seed fermenters, preferably one to three seed fermenters. In one embodiment the seed train comprises two seed fermenters. In another embodiment, the pre-culture comprises three seed fermenters. The volume of the fermentation medium present in the seed fermenters may increase within the seed train. For example, the volume of the second seed fermenter may be larger than the volume of the first seed fermenter. In one embodiment, the volume of the third seed fermenter is larger than the volume of the second seed fermenter and the volume of the second seed fermenter is larger than the volume of the first seed fermenter.

The pre-culture of Bacillus cells may be prepared by inoculating Bacillus cells producing the protein of interest from a frozen vial or a glycerol stock of into a first seed fermenter and the Bacillus cells are grown to a desired density. After the cultivation in the first seed fermenter, the Bacillus cells may be inoculated either directly into the production bioreactor, if no seed train is used, or into the next of a series of seed fermenters within a seed train.

Instead of using a vial containing the Bacillus cells producing the protein of interest, the pre-culture may also be prepared from Bacillus cells growing on an agar plate.

The criteria for the transfer of the Bacillus cells from one seed fermenter to a subsequent seed fermenter can be based on cultivation time (e.g. the Bacillus cells are transferred after a cultivation time of 12h to 40h) or reaching a certain value in an online signal e.g. oxygen uptake rate (OTR) (e.g. the Bacillus cells are transferred at an OTR between 30-180 mmol/(L*h)), or carbon dioxide evolution rate (CER) (e.g. the Bacillus cells are transferred at a CER between 40-200 mmol/(L*h)), or the period for which the feed solution has been added (e.g. the Bacillus cells are transferred after the feed solution has been added for 5-30h). In one embodiment, the Bacillus cells are transferred from one seed fermenter to a subsequent seed fermenter after culturing the Bacillus cells for 16 hours. In one embodiment a part of the fermentation broth present in the seed fermenter is transferred to another seed fermenter or the production bioreactor. In one embodiment the complete fermentation broth present in the seed fermenter is transferred to another seed fermenter or the production bioreactor.

The volume of the fermentation broth transferred from one seed fermenter to the next seed fermenter in a seed train is not less than 0.1% and not more than 20% of the initial volume of the fermentation medium present in the subsequent seed fermenter of the seed train. In one embodiment, the volume of the transferred fermentation broth of the first seed fermenter is between 0.1% and 15%, preferably between 1% and 15%, more preferably between 5% and 10%, most preferably between 8% and 10% of the volume of the fermentation medium present in the subsequent seed fermenter. In one embodiment the volume of the fermentation broth transferred from one seed fermenter to the next seed fermenter in a seed train is 0.1%, 0.2%, 0.3%, 0.4%, 0.5%, 0.6%, 0.7%, 0.8%, 0.9%, 1%, 2%, 3%, 4%, 5%, 6%, 7%, 8%, 9%, 10%, 11%, 12%, 13%, 14% or 15% of the volume of the fermentation medium present in the subsequent seed fermenter. In a preferred embodiment the volume of the fermentation broth transferred from one seed fermenter to the next seed fermenter in a seed train is 10% of the volume of the fermentation medium present in the subsequent seed fermenter.

According to the invention, the pre-culture is performed in fed-batch mode. If a seed train is used, at least the last stage of the seed train, i.e. the culture in the last seed fermenter before inoculation of the production bioreactor is performed in fed-batch mode. The fermentation process in the one or more preceding seed fermenters may be performed in batch mode, fed-batch mode, or continuous mode. Preferably, the fermentation process in the one or more seed fermenters preceding the last seed fermenter is performed in batch mode. Also preferably, the fermentation process in all seed fermenters preceding the last seed fermenter is performed in batch mode.

The fermentation medium and culture conditions used for pre-culture may be suitable for a rapid growth of the Bacillus cells. Therefore, the cells present in the pre-culture may initially be in lag phase, start to proliferate over time and preferably be in exponential phase at the time of the transfer into the next fermenter or the production bioreactor, respectively.

### Step b): inoculation of the inoculum into the production bioreactor

When the amount of Bacillus cells in the (last) seed fermenter of the pre-culture reaches its maximum, the fermentation broth present in this seed fermenter is transferred to the production bioreactor, in which the protein of interest is produced and inoculated into the fermentation medium.

The criteria for the transfer of the Bacillus cells from the (last) seed fermenter to the production bioreactor can be based on cultivation time (e.g. the Bacillus cells are transferred after a cultivation time of 12h to 40h) or reaching a certain value in an online signal e.g. oxygen uptake rate (OTR) (e.g. the Bacillus cells are transferred at an OTR between 30-180 mmol/(L*h)) or carbon dioxide evolution rate (CER) (e.g. the Bacillus cells are transferred at a CER between 40-200 mmol/(L*h)) or the period for which the feed solution has been added (e.g. the Bacillus cells are transferred after the feed solution has been added for 5-30h). In one embodiment, the Bacillus cells are transferred from the (last) seed fermenter to the production bioreactor after culturing the Bacillus cells for 22 hours in the (last) seed fermenter. In one embodiment a part of the fermentation broth present in the seed fermenter is transferred to another seed fermenter or the production bioreactor. In one embodiment the complete fermentation broth present in the seed fermenter is transferred to another seed fermenter or the production bioreactor.

The volume of the fermentation broth transferred from the (last) seed fermenter to the production bioreactor is not less than 0.1% and not more than 20% of the initial volume of the fermentation medium present in the production bioreactor. In one embodiment, the volume of the transferred fermentation broth of the (last) seed fermenter is between 0.1% and 15%, preferably between 1% and 15% or between 1% to 12%, more preferably between 1% and 10% or between 5% and 10%, most preferably between 8% and 10% of the volume of the fermentation medium present in the production bioreactor. In one embodiment the volume of the fermentation broth transferred from the (last) seed fermenter to the production bioreactor is 0.1%, 0.2%, 0.3%, 0.4%, 0.5%, 0.6%, 0.7%, 0.8%, 0.9%, 1%, 2%, 3%, 4%, 5%, 6%, 7%, 8%, 9%, 10%, 11%, 12%, 13%, 14% or 15% of the volume of the fermentation medium present in the production bioreactor. In one embodiment the volume of the fermentation broth transferred from the (last) seed fermenter to the production bioreactor is 10% of the volume of the fermentation medium present in the production bioreactor. In one embodiment the volume of the fermentation broth transferred from the (last) seed fermenter to the production bioreactor is 15% of the volume of the fermentation medium present in the production bioreactor.

### Step c): culturing in the production bioreactor

During culturing in the production bioreactor the majority of the protein of interest is produced. Hence, the amount of product produced in the production bioreactor is greater than the amount of product produced in the pre-culture. The amount of the protein of interest produced in the pre-culture can be determined by taking a sample of the pre-culture before the production bioreactor is inoculated with the cells from the pre-culture and determining the amount of the protein of interest in the sample from the pre-culture. The amount of product produced in the production bioreactor can be determined by taking a sample from the production bioreactor at the end of the culture and/or before harvesting the cells and determining the amount of the protein of interest in the sample from the production bioreactor. In one embodiment, the percentage of the amount of the protein of interest produced in pre-culture to the amount of protein of interest produced in the production bioreactor is not more than 10% or not more than 8%. In one embodiment, the percentage of the amount of the protein of interest produced in pre-culture to the amount of protein of interest produced in the production bioreactor is not more than 7%, not more than 6% or not more than 5%.

In the process of the present invention more than 200 g of a carbon source (preferably, a chemically defined carbon source) per liter of initial fermentation medium are added to the production bioreactor during culturing in the production bioreactor. Preferably, the total amount of a carbon source (preferably, a chemically defined carbon source) added to the production bioreactor during culturing in the production bioreactor is more than 300 g, more preferably more than 400 g per liter of initial fermentation medium. Preferably, at least 50% of the carbon source (preferably, a chemically defined carbon source) is provided in the fermentation process in the production bioreactor as feed solution, more preferred at least 60%, at least 70%, at least 80%, at least 90%, or 100% of the carbon source (preferably, a chemically defined carbon source) provided in the fermentation process is provided as feed solution in the fermentation process.

In one embodiment, the cultivation of the Bacillus cells in the production bioreactor may be performed in batch mode, fed-batch mode or continuous fermentation mode. In a preferred embodiment, the cultivation of the Bacillus cells in the production bioreactor is performed in fed-batch or continuous fermentation mode. Most preferably, the cultivation of the Bacillus cells in the production bioreactor is performed in fed-batch mode.

According to the invention, the Bacillus cells inoculated into the production bioreactor are cultured under conditions conducive for the production of the protein of interest. In one embodiment, the Bacillus cells inoculated into the production bioreactor are cultured under conditions conducive for the growth of the Bacillus cells and the production of the protein of interest. These conditions may be determined by the fermentation medium, the pH and/or the temperature used.

Fermentation processes can be performed with any media suitable for cell growth and production of the desired product for the particular Bacillus cell.

Bacillus cells are in general assumed to grow in a number of phases in fermentation, starting with a lag phase where the Bacillus cells adapt to the medium and start to grow, an exponential phase where the Bacillus cells grow at a constant growth rate providing an exponential increase in cell number and cell mass, a stationary phase, where the growth has stopped and the cell number remains constant and finally the death phase where the cell number decreases due to cell death. Preferably, the culture is stopped and the protein of interest is harvested before the death phase starts. Also preferably, the culture proceeds to the stationary phase before the protein of interest is harvested.

The medium and the culture conditions used in the production bioreactor may be suitable for optimal production of the protein of interest by the Bacillus cells. The culture conditions thus allow the cells present in the production fermenter to be in any growth phase suitable for the production of product.

### Fermentation medium

The fermentation medium for the seed fermenter may or may not be the same fermentation medium as used in the production bioreactor. Furthermore, the fermentation medium of the several seed fermenters in a seed train may be the same or different. The fermentation medium of the seed fermenter may be a chemically defined or a complex medium. The fermentation medium of the production bioreactor may be a chemically defined or a complex fermentation medium. In the seed fermenter run in fedbatch mode a complex medium or a chemically defined medium may be used. Preferably, the fermentation medium of the (last) seed fermenter is a chemically defined fermentation medium or a complex fermentation medium and the fermentation medium of the production bioreactor is a chemically defined fermentation medium. In one embodiment, the seed train comprises three seed fermenters and in the first seed fermenter a complex medium is used and in the second and third seed fermenters as well as in the production bioreactor a chemically defined medium is used. In one embodiment, the seed train comprises three seed fermenters and in the first and third seed fermenter a complex medium is used and in the second seed fermenter as well as in the production bioreactor a chemically defined medium is used. In one embodiment, the seed train comprises three seed fermenters and in the first, second and third seed fermenter a complex medium is used and in the production bioreactor a chemically defined medium is used. In one embodiment, the seed train comprises three seed fermenters and in the first, second and third seed fermenter a chemically defined medium is used and in the production bioreactor a chemically defined medium is used.

### Complex fermentation medium

The term "complex fermentation medium" refers to a fermentation medium that comprise a complex nutrient source in an amount of 0.5-30% w/v of the fermentation medium.

The term "complex nutrient source" is used herein for nutrient sources which are composed of chemically undefined compounds, i.e., compounds that are not known by their chemical formula, preferably comprising undefined organic nitrogen- and / or carbon-containing compounds. In contrast thereto, a "chemically defined nutrient source" (e.g., "chemically defined carbon source" or "chemically defined nitrogen source") is understood to be used for nutrient sources which are composed of chemically defined compounds. A "chemically defined component" is a component which is known by its chemical formula.

The term "complex nitrogen source" is used herein for a nutrient source that is composed of one or more chemically undefined nitrogen containing compounds, i.e., nitrogen containing compounds that are not known by their chemical formula, preferably comprising organic nitrogen containing compounds, e.g., proteins and/or amino acids with unknown composition.

The term "complex carbon source" is used herein for a carbon source that is composed of one or more chemically undefined carbon containing compounds, i.e., carbon containing compounds that are not known by their chemical formula, preferably comprising organic carbon containing compounds, e.g., carbohydrates with unknown composition.

It is clear for the skilled person that a complex nutrient source might be a mixture of different complex nutrient sources. Thus, a complex nitrogen source can comprise a complex carbon source and vice versa and a complex nitrogen source can be metabolized by the cells in a way that it functions as carbon source and vice versa.

In one embodiment, the complex nutrient source is a complex nitrogen source. Complex sources of nitrogen include, but are not limited to protein-containing substances, such as an extract from microbial, animal or plant cells, e.g., plant protein preparations, soy meal, corn meal, pea meal, corn gluten, cotton meal, peanut meal, potato meal, meat, casein, gelatins, whey, fish meal, yeast protein, yeast extract, tryptone, peptone, bacto-tryptone, bacto-peptone, wastes from the processing of microbial cells, plants, meat or animal bodies, and combinations thereof. In one embodiment, the complex nitrogen source is selected from the group consisting of plant protein, preferably potato protein, soy protein, corn protein, peanut, cotton protein, and/or pea protein, casein, tryptone, peptone and yeast extract and combinations thereof.

In one embodiment, the fermentation medium also comprises defined media components. In one embodiment, the fermentation medium also comprises a defined nitrogen source. Examples of inorganic nitrogen sources are ammonium, nitrate, and nitrit, and combinations thereof. In a preferred embodiment, the fermentation medium comprises a nitrogen source, wherein the nitrogen source is a complex or a defined nitrogen source or a combination thereof. In one embodiment, the defined nitrogen source is selected from the group consisting of ammonia, ammonium, ammonium salts, (e.g., ammonium chloride, ammonium nitrate, ammonium phosphate, ammonium sulfate, ammonium acetate), urea, nitrate, nitrate salts, nitrit, and amino acids, preferably, glutamate, and combinations thereof.

In one embodiment, the complex nutrient source is in an amount of 2-15% v/w of the fermentation medium. In another embodiment, the complex nutrient source is in an amount of 3-10% v/w of the fermentation medium.

In one embodiment, the complex fermentation medium further comprises a carbon source. The carbon source is preferably a complex or a defined carbon source or a combination thereof. In one embodiment, the complex nutrient source comprises a carbohydrate source. Various sugars and sugar-containing substances are suitable sources of carbon, and the sugars may be present in different stages of polymerisation. Preferred complex carbon sources to be used in the present invention are selected from the group consisting of molasse, corn steep liquor, cane sugar, dextrin, starch, starch hydrolysate, and cellulose hydrolysate, and combinations thereof. Preferred defined carbon sources are selected from the group consisting of carbohydrates, organic acids, and alcohols, preferably, glucose, fructose, galactose, xylose, arabinose, sucrose, maltose, lactose, acetic acid, propionic acid, lactic acid, formic acid, malic acid, citric acid, fumaric acid, glycerol, inositol, mannitol and sorbitol, and combinations thereof. Preferably, the defined carbon source is provided in form of a sirup, which can comprise up to 20%, preferably, up to 10%, more preferably up to 5% impurities. In one embodiment, the carbon source is sugar beet sirup, sugar cane sirup, corn sirup, preferably, high fructose corn sirup. In another embodiment, the complex carbon source is selected from the group consisting of molasses, corn steep liquor, dextrin, and starch, or combinations thereof, and wherein the defined carbon source is selected from the group consisting of glucose, fructose, galactose, xylose, arabinose, sucrose, maltose, dextrin, lactose, or combinations thereof.

In one embodiment, the fermentation medium is a complex medium comprising complex nitrogen and complex carbon sources. In one embodiment, the fermentation medium is a complex medium comprising complex nitrogen and carbon sources, wherein the complex nitrogen source may be partially hydrolyzed as described in WO 2004/003216.

In one embodiment, the fermentation medium also comprises a hydrogen source, an oxygen source, a sulfur source, a phosphorus source, a magnesium source, a sodium source, a potassium source, a trace element source, and a vitamin source as further described below.

### Chemically defined fermentation medium

The term "chemically defined fermentation medium" (also called herein "chemically defined medium", "defined medium", or "synthetic medium") is understood to be used for fermentation media which are essentially composed of chemically defined components in known concentrations. A "chemically defined component" is a component which is known by its chemical formula. A fermentation medium which is essentially composed of chemically defined component includes a medium which does not contain a complex nutrient source, in particular no complex carbon and/or complex nitrogen source, i.e., which does not contain complex raw materials having a chemically undefined composition. A fermentation medium which is essentially composed of chemically defined components may further include a medium which comprises an essentially small amount of a complex nutrient source, for instance a complex nitrogen and/or carbon source, an amount as defined below, which typically is not sufficient to maintain growth of the microorganism and/or to guarantee formation of a sufficient amount of biomass.

In that regard, complex raw materials have a chemically undefined composition due to the fact that, for instance, these raw materials contain many different compounds, among which complex heteropolymeric compounds, and have a variable composition due to seasonal variation and differences in geographical origin. Typical examples of complex raw materials functioning as a complex carbon and/or nitrogen source in fermentation are soybean meal, cotton seed meal, corn steep liquor, yeast extract, casein hydrolysate, molasses, and the like. An essentially small amount of a complex carbon and/or nitrogen source may be present in the chemically defined medium according to the invention, for instance as carry-over from the inoculum for the main fermentation. The inoculum for the main fermentation is not necessarily obtained by fermentation on a chemically defined medium. Most often, carry-over from the inoculum will be detectable through the presence of a small amount of a complex nitrogen source in the chemically defined medium of the main fermentation. Small amounts of a complex medium components, like complex carbon and/or nitrogen source, might also be introduced into the fermentation medium by the addition of small amounts of these complex components to the fermentation medium. It may be advantageous to use a complex carbon and/or nitrogen source in the fermentation process of the inoculum for the main fermentation, for instance to speed up the formation of biomass. i.e. to increase the growth rate of the microorganism, and/or to facilitate internal pH control. For the same reason, it may be advantageous to add an essentially small amount of a complex carbon and/or nitrogen source, e.g. yeast extract, to the initial stage of the main fermentation, especially to speed up biomass formation in the early stage of the fermentation process.

An essentially small amount of a complex nutrient source which may be added to the chemically defined fermentation medium in the fermentation process according to the invention is defined to be an amount of at the most 10% of the total amount of the respective nutrient, which is added in the fermentation process. In particular, an essentially small amount of a complex carbon and/or nitrogen source which may be added to the chemically defined fermentation medium is defined to be an amount of a complex carbon source resulting in at the most 10% of the total amount of carbon and/or an amount of a complex nitrogen source resulting in at the most 10% of the total amount of nitrogen, which is added in the fermentation process, preferably an amount of a complex carbon source resulting in at the most 5% of the total amount of carbon and/or an amount of a complex nitrogen source resulting in at the most 5% of the total amount of nitrogen, more preferably an amount of a complex carbon source resulting in at the most 1 % of the total amount of carbon and/or an amount of a complex nitrogen source resulting in at the most 1 % of the total amount of nitrogen, which is added in the fermentation process. Preferably, at the most 10% of the total amount of carbon and/or at the most 10% of the total amount of nitrogen, preferably an amount of at the most 5% of the total amount of carbon and/or an amount of at the most 5% of the total amount of nitrogen, more preferably an amount of at the most 1 % of the total amount of carbon and/or an amount of at the most 1 % of the total amount of nitrogen which is added in the fermentation process is added via carry-over from the inoculum. Most preferably, no complex carbon and/or complex nitrogen source is added to the fermentation medium in the fermentation process.

The term "chemically defined nutrient source" (e.g., "chemically defined carbon source" or "chemically defined nitrogen source") is understood to be used for nutrient sources which are composed of chemically defined compounds.

Culturing a microorganism in a chemically defined fermentation medium requires that cells be cultured in a medium which contain various chemically defined nutrient sources selected from the group consisting of chemically defined hydrogen source, chemically defined oxygen source, chemically defined carbon source, chemically defined nitrogen source, chemically defined sulfur source, chemically defined phosphorus source, chemically defined magnesium source, chemically defined sodium source, chemically defined potassium source, chemically defined trace element source, and chemically defined vitamin source.

Preferably, the chemically defined carbon source is selected from the group consisting of carbohydrates, organic acids, hydrocarbons, alcohols and mixtures thereof. Preferred carbohydrates are selected from the group consisting of glucose, fructose, galactose, xylose, arabinose, sucrose, maltose, maltotriose, lactose, dextrin, maltodextrins, starch and inulin, and mixtures thereof. Preferred alcohols are selected from the group consisting of glycerol, methanol and ethanol, inositol, mannitol and sorbitol and mixtures thereof. Preferred organic acids are selected from the group consisting of acetic acid, propionic acid, lactic acid, formic acid, malic acid, citric acid, fumaric acid and higher alkanoic acids and mixtures thereof. Preferably, the chemically defined carbon source comprises glucose or sucrose. More preferably, the chemically defined carbon source comprises glucose, even more preferably the predominant amount of the chemically defined carbon source is provided as glucose.

Most preferably, the chemically defined carbon source is glucose. It is to be understood that the chemically defined carbon source can be provided in form of a syrup, preferably as glucose syrup. As understood herein, the term "glucose" shall include glucose syrups. A glucose syrup is a viscous sugar solution with high sugar concentration. The sugars in glucose syrup are mainly glucose and to a minor extent also maltose and maltotriose in varying concentrations depending on the quality grade of the syrup. Preferably, besides glucose, maltose and maltotriose the syrup can comprise up to 10%, preferably, up to 5%, more preferably up to 3% impurities. Preferably, the glucose syrup is from corn.

The chemically defined nitrogen source is preferably selected from the group consisting of urea, ammonia, nitrate, nitrate salts, nitrit, ammonium salts such as ammonium chloride, ammonium sulphate, ammonium acetate, ammonium phosphate and ammonium nitrate, and amino acids such as glutamate or lysine and combinations thereof. More preferably, a chemically defined nitrogen source is selected from the group consisting of ammonia, ammonium sulphate and ammonium phosphate. Most preferably, the chemically defined nitrogen source is ammonia. The use of ammonia as a chemically defined nitrogen source has the advantage that ammonia additionally can function as a pH controlling agent.

Additional compounds can be added in complex and chemically defined fermentation medium as described below.

Oxygen is usually provided during the cultivation of the cells by aeration of the fermentation media by stirring or gassing. Hydrogen is usually provided due to the presence of water in the aqueous fermentation medium. However, hydrogen and oxygen are also contained within the carbon and/or nitrogen source and can be provided that way.

Magnesium can be provided to the fermentation medium by one or more magnesium salts, preferably selected from the group consisting of magnesium chloride, magnesium sulfate, magnesium nitrate, magnesium phosphate, and combinations thereof, or by magnesium hydroxide, or by combinations of one or more magnesium salts and magnesium hydroxide.

Sodium can be added to the fermentation medium by one or more sodium salts, preferably selected from the group consisting of sodium chloride, sodium nitrate, sodium sulphate, sodium phosphate, sodium hydroxide, and combinations thereof.

Calcium can be added to the fermentation medium by one or more calcium salts, preferably selected from the group consisting of calcium sulphate, calcium chloride, calcium nitrate, calcium phosphate, calcium hydroxide, and combination thereof.

Potassium can be added to the fermentation medium in chemically defined form by one or more potassium salts, preferably selected from the group consisting of potassium chloride, potassium nitrate, potassium sulphate, potassium phosphate, potassium hydroxide, and combination thereof.

Phosphorus can be added to the fermentation medium by one or more salts comprising phosphorus, preferably selected from the group consisting of potassium phosphate, sodium phosphate, magnesium phosphate, phosphoric acid, and combinations thereof. Preferably, at least 1 g of phosphorus is added per liter of initial fermentation medium.

Sulfur can be added to the fermentation medium by one or more salts comprising sulfur, preferably selected from the group consisting of potassium sulfate, sodium sulfate, magnesium sulfate, sulfuric acid, and combinations thereof.

Preferably, the fermentation medium comprises one or more selected from the group consisting of:
0.1 - 50 g nitrogen per liter of fermentation medium;
1 - 6 g phosphorus per liter of fermentation medium;
0.15 - 2 g sulfur per liter of fermentation medium;
0.4 - 8 g potassium per liter of fermentation medium;
0.1 - 2 g sodium per liter of fermentation medium;
0.01 - 3 g calcium per liter of fermentation medium; and
0.1 - 10 g magnesium per liter of fermentation medium.

One or more trace element ions can be added to the fermentation medium, preferably in amounts of below 10 mmol/L initial fermentation medium each. These trace element ions are selected from the group consisting of iron, copper, manganese, zinc, cobalt, nickel, molybdenum, selenium, and boron and combinations thereof. Preferably, the trace element ions iron, copper, manganese, zinc, cobalt, nickel, and molybdenum are added to the fermentation medium. Preferably, the one or more trace element ions are added to the fermentation medium in an amount selected from the group consisting of 50 µmol to 5 mmol per liter of initial medium of iron, 40 µmol to 4 mmol per liter of initial medium copper, 30 µmol to 3 mmol per liter of initial medium manganese, 20 µmol to 2 mmol per liter of initial medium zinc, 1 µmol to 100 µmol per liter of initial medium cobalt, 2 µmol to 200 µmol per liter of initial medium nickel, and 0.3 µmol to 30 µmol per liter of initial medium molybdenum, and combinations thereof. For adding each trace element preferably one or more from the group consisting of chloride, phosphate, sulphate, nitrate, citrate and acetate salts can be used.

Compounds which may optionally be included in the fermentation medium are chelating agents, such as citric acid, MGDA, NTA, or GLDA, and buffering agents such as mono- and dipotassium phosphate, calcium carbonate, and the like. Buffering agents preferably are added when dealing with processes without an external pH control. In addition, an antifoaming agent may be dosed prior to and/or during the fermentation process.

Vitamins refer to a group of structurally unrelated organic compounds, which are necessary for the normal metabolism of cells. Cells are known to vary widely in their ability to synthesize the vitamins they require. A vitamin should be added to the fermentation medium of Bacillus cells not capable of synthesizing said vitamin. Vitamins can be selected from the group of thiamin, riboflavin, pyridoxal, nicotinic acid or nicotinamide, pantothenic acid, cyanocobalamin, folic acid, biotin, lipoic acid, purines, pyrimidines, inositol, choline and hemins.

Preferably, the fermentation medium also comprises a selection agent, e.g., an antibiotic, such as ampicillin, tetracycline, kanamycin, hygromycin, bleomycin, chloroamphenicol, streptomycin or phleomycin, to which the selectable marker of the cells provides resistance.

The amount of necessary compounds to be added to the medium will mainly depend on the amount of biomass which is to be formed in the fermentation process. The amount of biomass formed may vary widely, typically the amount of biomass is from about 10 to about 150 grams of dry cell mass per liter of fermentation broth. Usually, for protein production, fermentations producing an amount of biomass which is lower than about 10 g of dry cell mass per liter of fermentation broth are not considered industrially relevant.

The optimum amount of each component of a defined medium, as well as which compounds are essential and which are non-essential, will depend on the type of Bacillus cell which is subjected to fermentation in a medium, on the amount of biomass and on the product to be formed. Typically, the amount of medium components necessary for growth of the Bacillus cell may be determined in relation to the amount of carbon source used in the fermentation, since the amount of biomass formed will be primarily determined by the amount of carbon source used.

A feed medium or feed solution used e.g. when the culture is run in fed-batch mode may be any of the above mentioned medium components or combination thereof. It is understood herein that at least part of the compounds that are provided as feed solution can already be present to a certain extent in the fermentation medium prior to feeding of said compounds. In one embodiment the feed solution comprises glucose. In one embodiment, the feed solution comprises 40% to 60% glucose, preferably 42% to 58% glucose, more preferably 45% to 55% glucose, even more preferably 47% to 52% glucose and most preferably 50% glucose. In one embodiment, the same feed solution may be used for the seed fermenter run in fedbatch mode and the production bioreactor. In one embodiment, the feed solution used for the seed fermenter run in fedbatch mode differs from the feed solution used in the production bioreactor. In one embodiment, the feed solution used for the seed fermenter run in fedbatch mode and the feed solution used in the production bioreactor has the same concentration of glucose, but the feed solution used in the production bioreactor contains salts which are not present in the feed solution used for the seed fermenter run in fedbatch mode.

Various feed profiles are known in the art. A feed solution can be added continuously or discontinuously during the fermentation process. Discontinuous addition of a feed solution can occur once during the fermentation process as a single bolus or several times with different or same volumes. Continuous addition of a feed solution can occur during the fermentation process at the same or at varying rates (i.e., volume per time). Also combinations of continuous and discontinuous feeding profiles can be applied during the fermentation process. Components of the fermentation medium that are provided as feed solution can be added in one feed solution or as different feed solutions. In case more than one feed solution is applied, the feed solutions can have the same or different feed profiles as described above.

Preferably, prior to inoculation the fermentation medium and feed solutions are sterilized in order to prevent or reduce growth of microorganisms during the fermentation process, which are different from the inoculated microbial cells. Sterilization can be performed with methods known in the art, for example but not limited to, autoclaving or sterile filtration. Some or all medium components can be sterilized separately from other medium components to avoid interactions of medium components during sterilization treatment or to avoid decomposition of medium components under sterilization conditions.

### pH and temperature of fermentation

The pH, temperature, antifoam or other specific fermentation conditions may be applied according to standard conditions known in the art. In one embodiment, the fermentation conditions are adjusted to obtain maximum yields of the protein of interest.

Preferably, the Bacillus cells are cultured at a temperature of 25°C to 45°C, preferably of 27°C to 40°C, more preferably of 27°C to 37°C, most preferably at a temperature of 37°C. Moreover, a temperature shift may be applied, wherein the culture temperature is lowered, e.g. from 37°C to a temperature of 33°C, 32°C, 31°C, 30°C, 29°C or 28°C.

Depending on the Bacillus cell, the pH of the fermentation broth during cultivation is adjusted. Preferably, the pH of the chemically defined medium is adjusted prior to inoculation. Preferably, the pH of the chemically defined medium is adjusted prior to inoculation, but after sterilization. Preferably, the pH of the chemically defined medium is adjusted prior to inoculation to pH 6.6 to 9, preferably to pH 6.6 to 8.5, more preferably to pH 6.8 to 8.5, most preferably to pH 6.8 to pH 8.0. As an example, the pH is adjusted to or above pH 6.8, pH 7.0, pH 7.2, pH 7.4, or pH 7.6. Preferably, the pH of the fermentation broth during cultivation of the *Bacillus* cells is adjusted to pH 6.8 to 9, preferably to pH 6.8 to 8.5, more preferably to pH 7.0 to 8.5, most preferably to pH 7.2 to pH 8.0.

In one embodiment, fermentation is carried out with stirring and/or shaking the fermentation medium. In a specific embodiment, fermentation is carried out with stirring the fermentation medium with 50 - 2000 rpm, preferably with 50 - 1600 rpm, further preferred with 800 - 1400 rpm, more preferably with 50 - 200 rpm.

In one embodiment, oxygen is added to the fermentation medium during cultivation, preferably by stirring and/or agitation as described herein or by gassing, preferably with 0-3 bar air or oxygen. In one embodiment, fermentation is performed under saturation with oxygen.

Culture conditions for a given cell type may also be found in the scientific literature and/or from the source of the cell such as the American Type Culture Collection (ATCC) and Fungal Genetics Stock Center.

### Time to harvest

The time to harvest is the time span measured from the inoculation of the production bioreactor with the Bacillus cells to the time point when the concentration of the protein of interest has reached a predetermined level and the fermentation broth is harvested. In an embodiment, the protein is harvested when its concentration is at least 5 g protein/kg fermentation broth, more preferably at least 7.5 g protein/kg fermentation broth, most preferably at least 10 g protein/kg fermentation broth. In a preferred embodiment, the protein is harvested when its concentration is 10 g protein/kg fermentation broth.

Therefore, in one embodiment, the invention refers to a process for culturing Bacillus cells producing a protein of interest, comprising the steps of:
(a) providing a pre-culture of said Bacillus cells;
(b) inoculating a production bioreactor with the pre-culture of step (a); and
(c) culturing the Bacillus cells in the production bioreactor under conditions conducive for the production of the protein of interest,
wherein the pre-culture of step (a) is performed in fed-batch mode, and
wherein the Bacillus cells are cells of Bacillus subtilis, Bacillus pumilus, Bacillus licheniformis, or Bacillus lentus and
wherein the process further comprises a step (d) of harvesting the protein when the concentration of the protein of interest in the fermentation broth is at least 10g protein/kg fermentation broth.

The concentration of the protein of interest in the fermentation broth may be monitored by continuously measuring the concentration in samples from the fermentation. If the protein of interest is an enzyme, enzyme activity assays may be performed which then provide information on the concentration of the enzyme in the sample. For example, a protease activity assay may involve succinyl - Ala - Ala - Pro - Phe - p-nitroanilide (Suc-AAPF-pNA, short: AAPF) as substrate. An amylase activity assay may employ the substrate Ethyliden-4-nitrophenyl-α-D-maltoheptaosid (EPS) as substrate. Kits containing EPS substrate and alpha-glucosidase are available from Roche Costum Biotech (cat. No. 10880078103) and are described in Lorentz K. et al. (2000) Clin. Chem. 46(5): 644 - 649.

The time to harvest is dependent on the protein produced by the Bacillus cells and may thus vary. However, when applying the process of the invention comprising a pre-culture step performed in fed-batch mode, the time until harvest is reduced by 5% to 30%, preferably by 10 to 30%, most preferably by 15 to 30% compared to a process where the pre-culture step of performed in batch mode. In one embodiment, the fermentation time in the production bioreactor is reduced by at least 5%, preferably by at least 10%, more preferably by at least 15%, more preferably by at least 20%, more preferably by at least 25% and most preferably by at least 30% compared to a process where the pre-culture step of performed in batch mode.

### Bacillus cells

The Bacillus cell is a *Bacillus* cell of *Bacillus subtilis, Bacillus pumilus, Bacillus licheniformis,* or *Bacillus lentus.* Preferably, the cell is a *Bacillus licheniformis* cell.

In one embodiment, the cell comprises one or more genetic constructs for heterologous gene expression, i.e. is genetically modified to express a protein. Bacillus cells genetically modified to express a protein are also called "recombinant" or "transgenic" Bacillus cells. They express a recombinant protein. The recombinant protein may be the protein or interest.

### Protein of interest

The present invention relates to a process for culturing Bacillus cells in order to produce a protein of interest.

### Proteins

There is no limitation on the origin of the protein of interest produced according to the method of the invention. Thus, the term protein of interest includes not only natural or wild-type proteins, but also any mutants, variants, fragments, etc. of the protein of interest, as well as a synthetic protein. Such genetically engineered proteins can be prepared as is generally known in the art, e.g., by site-directed mutagenesis, by PCR (using a PCR fragment containing the desired mutation as one of the primers in the PCR reactions), or by random mutagenesis.

The Bacillus cell can comprise the gene encoding the protein of interest (i.e., gene of interest) endogenously, i.e. the Bacillus cell is not recombinant, or the gene of interest can be heterologous to the Bacillus cell, i.e. the Bacillus cell is recombinant. Preferably, the gene encoding the protein of interest is heterologous to the Bacillus cell.

The desired product may be secreted into the liquid fraction of the fermentation broth or may remain inside the Bacillus cells. In a preferred embodiment, the fermentation product is secreted by the microorganism into the fermentation broth. Secretion of the protein of interest into the fermentation medium allows for separation of the protein of interest from the Bacillus cells. For secretion of a protein into the fermentation medium the nucleic acid construct used for expressing the protein of interest may comprise a polynucleotide coding for a signal peptide that directs secretion of the protein of interest into the fermentation medium. Various signal peptides are known in the art. Preferred signal peptides are selected from the group consisting of the signal peptide of the AprE protein from *Bacillus subtilis* or the signal peptide from the YvcE protein from *Bacillus subitilis.*

In a more preferred embodiment, the protein of interest is an enzyme.

In a preferred embodiment, the enzyme is selected from the group consisting of hydrolases, oxidases, isomerases, e.g. amylase, alpha-amylase, glucoamylase, pullulanase, protease, metalloprotease, peptidase, lipase, cutinase, acyl transferase, cellulase, endoglucanase, glucosidase, cellubiohydrolase, xylanase, xyloglucantransferase, xylosidase, mannanase, phytase, phosphatase, xylose isomerase, glucose isomerase, lactase, acetolactate decarboxylase, pectinase, pectin methylesterase, polygalacturonidase, lyase, pectate lyase, arabinase, arabinofuranosidase, galactanase, laccase, peroxidase and asparaginase, preferably wherein the enzyme is an amylase or protease.

In a particular preferred embodiment, the following enzymes are preferred:

### Protease

Enzymes having proteolytic activity are called "proteases" or "peptidases". Proteases are active proteins exerting "protease activity" or "proteolytic activity".

Proteases are members of class EC 3.4. Proteases include aminopeptidases (EC 3.4.11), dipeptidases (EC 3.4.13), dipeptidyl-peptidases and tripeptidyl-peptidases (EC 3.4.14), peptidyl-dipeptidases (EC 3.4.15), serine-type carboxypeptidases (EC 3.4.16), metallocarboxypeptidases (EC 3.4.17), cysteine-type carboxypeptidases (EC 3.4.18), omega peptidases (EC 3.4.19), serine endopeptidases (EC 3.4.21), cysteine endopeptidases (EC 3.4.22), aspartic endopeptidases (EC 3.4.23), metallo-endopeptidases (EC 3.4.24), threonine endopeptidases (EC 3.4.25), endopeptidases of unknown catalytic mechanism (EC 3.4.99).

Commercially available protease enzymes include, but are not limited, to Lavergy^{™} Pro (BASF); Alcalase^{®}, Blaze^{®}, Duralase^{™}, Durazym^{™}, Relase^{®}, Relase^{®} Ultra, Savinase^{®}, Savinase^{®} Ultra, Primase^{®}, Polarzyme^{®}, Kannase^{®}, Liquanase^{®}, Liquanase^{®} Ultra, Ovozyme^{®}, Coronase^{®}, Coronase^{®} Ultra, Neutrase^{®}, Everlase^{®} and Esperase^{®} (Novozymes A/S), those sold under the tradename Maxatase^{®}, Maxacal^{®}, Maxapem^{®}, Purafect^{®}, Purafect^{®} Prime, Purafect MA^{®}, Purafect Ox^{®}, Purafect OxP^{®}, Puramax^{®}, Properase^{®}, FN2^{®}, FN3^{®}, FN4^{®}, Excellase^{®}, Eraser^{®}, Ultimase^{®}, Opticlean^{®}, Effectenz^{®}, Preferenz^{®} and Optimase^{®} (Danisco/DuPont), Axapem^{™} (Gist-Brocases N.V.), *Bacillus lentus* Alkaline Protease, and KAP (*Bacillus alkalophilus* subtilisin) from Kao.

In one embodiment the protease may be selected from serine proteases (EC 3.4.21). Serine proteases or serine peptidases (EC 3.4.21) are characterized by having a serine in the catalytically active site, which forms a covalent adduct with the substrate during the catalytic reaction. A serine protease may be selected from the group consisting of chymotrypsin (e.g., EC 3.4.21.1), elastase (e.g., EC 3.4.21.36, EC 3.4.21.37 or EC 3.4.21.71), granzyme (e.g., EC 3.4.21.78 or EC 3.4.21.79), kallikrein (e.g., EC 3.4.21.34, EC 3.4.21.35, EC 3.4.21.118, or EC 3.4.21.119,) plasmin (e.g., EC 3.4.21.7), trypsin (e.g., EC 3.4.21.4), thrombin (e.g., EC 3.4.21.5,) and subtilisin (also known as subtilopeptidase, e.g., EC 3.4.21.62), the latter hereinafter also being referred to as "subtilisin".

A sub-group of the serine proteases tentatively designated subtilases has been proposed by Siezen et al. (1991), Protein Eng. 4:719-737 and Siezen et al. (1997), Protein Science 6:501-523. They are defined by homology analysis of more than 170 amino acid sequences of serine proteases previously referred to as subtilisin-like proteases. A subtilisin was previously often defined as a serine protease produced by Gram-positive bacteria or fungi, and according to Siezen et al. now is a subgroup of the subtilases. A wide variety of subtilases have been identified, and the amino acid sequence of a number of subtilases has been determined. For a more detailed description of such subtilases and their amino acid sequences reference is made to Siezen et al. (1997), Protein Science 6:501-523.

The subtilases may be divided into 6 sub-divisions, i.e. the subtilisin family, thermitase family, the proteinase K family, the lantibiotic peptidase family, the kexin family and the pyrolysin family.

A subgroup of the subtilases are the subtilisins, which are serine proteases from the family S8 as defined by the MEROPS database (merops.sanger.ac.uk). Peptidase family S8 contains the serine endopeptidase subtilisin and its homologues. In subfamily S8A, the active site residues frequently occur in the motifs Asp-Thr/Ser-Gly, His-Gly-Thr-His and Gly-Thr-Ser-Met-Ala-Xaa-Pro. Most members of the peptidase family S8 are active at neutral-mildly alkali pH. Many peptidases in the family are thermostable.

Prominent members of family S8, subfamily A are:

| name | MEROPS Family S8, Subfamily A |
|---|---|
| Subtilisin Carlsberg | S08.001 |
| Subtilisin lentus | S08.003 |
| Thermitase | S08.007 |
| Subtilisin BPN' | S08.034 |
| Subtilisin DY | S08.037 |
| Alkaline peptidase | S08.038 |
| Subtilisin ALP 1 | S08.045 |
| Subtilisin sendai | S08.098 |
| Alkaline elastase YaB | S08.157 |

Proteases of the subtilisin type (EC 3.4.21.62) and variants may be bacterial proteases. Said bacterial protease may be from a Gram-positive bacterium such as *Bacillus, Clostridium, Enterococcus, Geobacillus, Lactobacillus, Lactococcus, Oceanobacillus, Staphylococcus, Streptococcus,* or *Streptomyces,* or a Gram-negative bacterium such as a *Campylobacter,* E. *coli, Flavobacterium, Fusobacterium, Helicobacter, Ilyobacter, Neisseria, Pseudomonas, Salmonella,* or *Ureaplasma.* A review of this family is provided, for example, in "Subtilases: Subtilisin-like Proteases" by R. Siezen, pages 75-95 in "Subtilisin enzymes", edited by R. Bott and C. Betzel, New York, 1996.

At least one protease may be selected from the following: subtilisin from *Bacillus amyloliquefaciens* BPN' (described by Vasantha et al. (1984) J. Bacteriol. Volume 159, p. 811-819 and JA Wells et al. (1983) in Nucleic Acids Research, Volume 11, p. 7911-7925); subtilisin from *Bacillus licheniformis* (subtilisin Carlsberg; disclosed in EL Smith et al. (1968) in J. Biol Chem, Volume 243, pp. 2184-2191, and Jacobs et al. (1985) in Nucl. Acids Res, Vol 13, p. 8913-8926); subtilisin PB92 (original sequence of the alkaline protease PB92 is described in EP 283075 A2); subtilisin 147 and/or 309 (Esperase^{®}, Savinase^{®}, respectively) as disclosed in WO 89/06279; subtilisin from *Bacillus lentus* as disclosed in WO 91/02792, such as from *Bacillus lentus* DSM 5483 or the variants of *Bacillus lentus* DSM 5483 as described in WO 95/23221; subtilisin from *Bacillus alcalophilus* (DSM 11233) disclosed in DE 10064983; subtilisin from *Bacillus gibsonii* (*DSM* 14391) as disclosed in WO 2003/054184; subtilisin from *Bacillus sp.* (DSM 14390) disclosed in WO 2003/056017; subtilisin from *Bacillus sp.* (DSM 14392) disclosed in WO 2003/055974; subtilisin from *Bacillus gibsonii* (DSM 14393) disclosed in WO 2003/054184; subtilisin having SEQ ID NO: 4 as described in WO 2005/063974; subtilisin having SEQ ID NO: 4 as described in WO 2005/103244; subtilisin having SEQ ID NO: 7 as described in WO 2005/103244; and subtilisin having SEQ ID NO: 2 as described in application DE 102005028295.4.

Proteases also include the variants described in: WO 92/19729, WO 95/23221, WO 96/34946, WO 98/20115, WO 98/20116, WO 99/11768, WO 01/44452, WO 02/088340, WO 03/006602, WO 2004/03186, WO 2004/041979, WO 2007/006305, WO 2011/036263, WO 2011/036264, and WO 2011/072099. Suitable examples comprise especially protease variants of subtilisin protease derived from SEQ ID NO:22 as described in EP 1 921 147 with amino acid substitutions in one or more of the following positions: 3, 4, 9, 15, 24, 27, 33, 36, 57, 68, 76, 77, 87, 95, 96, 97, 98, 99, 100, 101, 102, 103, 104, 106, 118, 120, 123, 128, 129, 130, 131, 154, 160, 167, 170, 194, 195, 199, 205, 206, 217, 218, 222, 224, 232, 235, 236, 245, 248, 252 and 274 which have proteolytic activity. In addition, a subtilisin protease is not mutated at positions Asp32, His64 and Ser221.

A subtilisin-like enzyme may have SEQ ID NO:22 as described in EP 1921147, or may be a variant thereof which is at least 80%, at least 90%, at least 95% or at least 98% identical to SEQ ID NO:22 as described in EP 1 921 147 and has proteolytic activity. In one embodiment, a subtilisin-like enzyme is at least 80%, at least 90%, at least 95% or at least 98% identical to SEQ ID NO:22 as described in EP 1 921 147 and is characterized by having amino acid glutamic acid (E), or aspartic acid (D), or asparagine (N), or glutamine (Q), or alanine (A), or glycine (G), or serine (S) at position 101 (according to BPN' numbering) and has proteolytic activity. In one embodiment, a subtilisin-like enzyme is at least 80%, at least 90%, at least 95% or at least 98% identical to SEQ ID NO:22 as described in EP 1 921 147 and is characterized by having amino acid glutamic acid (E), or aspartic acid (D), at position 101 (according to BPN' numbering) and has proteolytic activity. Such a subtilisin variant may comprise an amino acid substitution at position 101, such as R101E or R101D, alone or in combination with one or more substitutions at positions 3, 4, 9, 15, 24, 27, 33, 36, 57, 68, 76, 77, 87, 95, 96, 97, 98, 99, 100, 101, 102, 103, 104, 106, 118, 120, 123, 128, 129, 130, 131, 154, 160, 167, 170, 194, 195, 199, 205, 206, 217, 218, 222, 224, 232, 235, 236, 245, 248, 252 and/or 274 (according to BPN' numbering) and has proteolytic activity. In one embodiment, said protease comprises one or more further substitutions (a) to (h): (a) threonine at position 3 (3T), (b) isoleucine at position 4 (4I), (c) alanine, threonine or arginine at position 63 (63A, 63T, or 63R), (d) aspartic acid or glutamic acid at position 156 (156D or 156E), (e) proline at position 194 (194P), (f) methionine at position 199 (199M), (g) isoleucine at position 205 (205I), (h) aspartic acid, glutamic acid or glycine at position 217 (217D, 217E or 217G), (i) combinations of two or more amino acids according to (a) to (h).

A subtilisin-like enzyme may have an amino acid sequence being at least 80% identical to SEQ ID NO:22 as described in EP 1 921 147 and being further characterized by comprising the substitution R101E, and one or more substitutions selected from the group consisting of S156D, L262E, Q137H, S3T, R45E,D,Q, P55N, T58W,Y,L, Q59D,M,N,T, G61 D,R, S87E, G97S, A98D,E,R, S106A,W, N117E, H120V,D,K,N, S125M, P129D, E136Q, S144W, S161T, S163A,G, Y171 L, A172S, N185Q, V199M, Y209W, M222Q, N238H, V244T, N261T,D and L262N,Q,D (as described in WO 2016/096711 and according to the BPN' numbering), and has proteolytic activity.

Proteases used in the present invention have proteolytic activity. The methods for determining proteolytic activity are well-known in the literature (see e.g. Gupta et al. (2002), Appl. Microbiol. Biotechnol. 60: 381-395). Proteolytic activity may be determined by using Succinyl-Ala-Ala-Pro-Phe-p-nitroanilide (Suc-AAPF-pNA, short AAPF; see e.g. DelMar et al. (1979), Analytical Biochem 99, 316-320) as substrate. pNA is cleaved from the substrate molecule by proteolytic cleavage, resulting in release of yellow color of free pNA which can be quantified by measuring OD405.

### Amylase

Alpha-amylase (E.C. 3.2.1.1) enzymes may perform endohydrolysis of (1->4)-alpha-D-glucosidic linkages in polysaccharides containing three or more (1->4)-alpha-linked D-glucose units. Amylase enzymes act on starch, glycogen and related polysaccharides and oligosaccharides in a random manner; reducing groups are liberated in the alpha-configuration. Other examples of amylase enzymes include: Beta-amylase (E.C. 3.2.1.2), Glucan 1,4-alpha-maltotetraohydrolase (E.C. 3.2.1.60), Isoamylase (E.C. 3.2.1.68), Glucan 1,4-alpha-maltohexaosidase (E.C. 3.2.1.98), and Glucan 1,4-alpha-maltohydrolase (E.C. 3.2.1.133).

Amylase enzymes have been described in patent documents including, but not limited to: WO 2002/068589, WO 2002/068597, WO 2003/083054, WO 2004/091544, and WO 2008/080093.

An amylase derived from *Bacillus licheniformis* has SEQ ID NO:2 as described in WO 95/10603. Suitable variants of this amylase are those which are at least 90% identical to SEQ ID NO: 2 as described in WO 95/10603 and/or comprise one or more substitutions in the following positions: 15, 23, 105, 106, 124, 128, 133, 154, 156, 178, 179, 181, 188, 190, 197, 201, 202, 207, 208, 209, 211, 243, 264, 304, 305, 391, 408, and 444 and have amylolytic activity. Such variants are described in WO 94/02597, WO 94/018314, WO 97/043424 and SEQ ID NO:4 of WO 99/019467.

An amylase derived from *B. stearothermophilus* has SEQ ID NO:6 as described in WO 02/10355. Suitable variants of this amylase are those which are at least 90% identical thereto and have amylolytic activity. Suitable variants of SEQ ID NO:6 include those which are at least 90% identical to SEQ ID NO:6 as described in WO 02/10355 and/or further comprise a deletion in positions 181 and/or 182 and/or a substitution in position 193.

An amylase derived from Bacillus sp.707 has SEQ ID NO:6 as disclosed in WO 99/19467 or is at least 90% identical thereto having amylolytic activity.

An amylase derived from *Bacillus halmapalus* has SEQ ID NO:2 or SEQ ID NO:7 as described in WO 96/23872, also described as SP-722, or is at least 90% identical to one of the sequences which has amylolytic activity.

An amylase derived from Bacillus sp. DSM 12649 has SEQ ID NO:4 as disclosed in WO 00/22103 or is at least 90% identical thereto having amylolytic activity.

An amylase derived from Bacillus strain TS-23 has SEQ ID NO:2 as disclosed in WO 2009/061380 or is at least 90 % identical thereto having amylolytic activity.

An amylase derived from Cytophaga sp. has SEQ ID NO:1 as disclosed in WO 2013/184577 or is at least 90% identical thereto having amylolytic activity.

An amylase derived from *Bacillus megaterium* DSM 90 has SEQ ID NO:1 as disclosed in WO 2010/104675 or is at least 90% identical thereto having amylolytic activity.

Amylases are known having amino acids 1 to 485 of SEQ ID NO:2 as described in WO 00/60060 or amylases comprising an amino acid sequence which is at least 96% identical to amino acids 1 to 485 of SEQ ID NO:2 which have amylolytic activity.

Amylases are also known having SEQ ID NO: 12 as described in WO 2006/002643 or amylases having at least 80% identity thereto and have amylolytic activity. Suitable amylases include those having at least 80% identity compared to SEQ ID NO:12 and/or comprising the substitutions at positions Y295F and M202LITV and have amylolytic activity.

Amylases are also known having SEQ ID NO:6 as described in WO 2011/098531 or amylases having at least 80% identity thereto having amylolytic activity. Suitable amylases include those having at least 80% identity compared to SEQ ID NO:6 and/or comprising a substitution at one or more positions selected from the group consisting of 193 [G,A,S,T or M], 195 [F,W,Y,L,I or V], 197 [F,W,Y,L,I or V], 198 [Q or N], 200 [F,W,Y,L,I or V], 203 [F,W,Y,L,I or V], 206 [F,W,Y,N,L,I,V,H,Q,D or E], 210 [F,W,Y,L,I or V], 212 [F,W,Y,L,I or V], 213 [G,A,S,T or M] and 243 [F,W,Y,L,I or V] and have amylolytic activity.

Amylases are known having SEQ ID NO:1 as described in WO 2013/001078 or amylases having at least 85% identity thereto having amylolytic activity. Suitable amylases include those having at least 85% identity compared to SEQ ID NO:1 and/or comprising an alteration at two or more (several) positions corresponding to positions G304, W140, W189, D134, E260, F262, W284, W347, W439, W469, G476, and G477 and having amylolytic activity.

Amylases are known having SEQ ID NO:2 as described in WO 2013/001087 or amylases having at least 85% identity thereto and having amylolytic activity. Suitable amylases include those having at least 85% identity compared to SEQ ID NO:2 and/or comprising a deletion of positions 181+182, or 182+183, or 183+184, which have amylolytic activity. Suitable amylases include those having at least 85% identity compared to SEQ ID NO:2 and/or comprising a deletion of positions 181+182, or 182+183, or 183+184, which comprise one or two or more modifications in any of positions corresponding to W140, W159, W167, Q169, W189, E194, N260, F262, W284, F289, G304, G305, R320, W347, W439, W469, G476 and G477 and have amylolytic activity.

Amylases also include hybrid α-amylases of the above mentioned amylases as for example as described in WO 2006/066594.

Commercially available amylase enzymes include: Amplify^{®}, Duramyl^{™}, Termamyl^{™}, Fungamyl^{™}, Stainzyme^{™}, Stainzyme Plus^{™}, Natalase^{™}, Liquozyme Xand BAN^{™} (from Novozymes A/S), and Rapidase^{™}, Purastar^{™}, PoweraseTM, Effectenz^{™} (M100 from DuPont), Preferenz^{™} (S1000, S110 and F1000; from DuPont), PrimaGreen^{™} (ALL; DuPont), Optisize^{™} (DuPont).

In one embodiment, the enzyme is a Termamyl-like amylase. In the present context, the term "Termamyl-like enzyme" is intended to indicate an α-amylase, which, at the amino acid level, has a sequence identity of at least 60% to the *B. licheniformis* α-amylase described in WO 96/23874. In an embodiment, the Termamyl-like α-amylase displays at least 70%, 75%, 80%, 85%, 90%, 95%, 97%, 99% identity to the *B. licheniformis* α-amylase described in WO 96/23874.

Another α-amylase herein is Natalase or a variant thereof as described in WO 95/26397, WO 99/19467 and WO 01/66712.

### Lipase

"Lipase", "lipolytic enzyme", "lipid esterase", all refer to an enzyme of EC class 3.1.1 ("carboxylic ester hydrolase"). Lipases (E.C. 3.1.1.3, Triacylglycerol lipase) may hydrolyze triglycerides to more hydrophilic mono- and diglycerides, free fatty acids, and glycerol. Lipase enzymes usually includes also enzymes which are active on substrates different from triglycerides or cleave specific fatty acids, such as Phospholipase A (E.C. 3.1.1.4), Galactolipase (E.C. 3.1.1.26), cutinase (EC 3.1.1.74), and enzymes having sterol esterase activity (EC 3.1.1.13) and/or wax-ester hydrolase activity (EC 3.1.1.50).

Many lipase enzymes have been described in the prior art, including, but not being limited to: WO 00/032758, WO 03/089620, WO 2005/032496, WO 2005/086900, WO 2006/00976, WO 2006/031699, WO 2008/036863, WO 2011/046812, and WO 2014/059360.

### Cellulase

"Cellulases", "cellulase enzymes" or "cellulolytic enzymes" are enzymes involved in the hydrolysis of cellulose. Three major types of cellulases are known, namely endo-beta-1,4-glucanase (endo-1,4-P-D-glucan 4-glucanohydrolase, E.C. 3.2.1.4; hydrolyzing β-1,4-glucosidic bonds in cellulose), cellobiohydrolase (1,4-P-D-glucan cellobiohydrolase, EC 3.2.1.91), and beta-glucosidase (EC 3.2.1.21).

Cellulase enzymes have been described in patents and published patent applications including, but not limited to: WO 97/025417, WO 98/024799, WO 03/068910, WO 2005/003319, and WO 2009/020459.

Commercially available cellulase enzymes include Celluzyme^{™}, Endolase^{™}, Carezyme^{™}, Cellusoft^{™}, Renozyme^{™}, Celluclean^{™} (from Novozymes A/S), Ecostone^{™}, Biotouch^{™}, Econase^{™}, Ecopulp^{™} (from AB Enzymes Finland), Clazinase^{™}, and Puradax HA^{™}, Genencor detergent cellulase L, IndiAge^{™} Neutra (from Genencor International Inc./DuPont), Revitalenz^{™} (2000 from DuPont), Primafast^{™} (DuPont) and KAC-500^{™} (from Kao Corporation).

Cellulases used in the methods according to the invention have "cellulolytic activity" or "cellulase activity". Assays for measurement of cellulolytic activity are known to those skilled in the art. For example, cellulolytic activity may be determined by virtue of the fact that cellulase hydrolyses carboxymethyl cellulose to reducing carbohydrates, the reducing ability of which is determined colorimetrically by means of the ferricyanide reaction, according to Hoffman, W. S., J. Biol. Chem. 120, 51 (1937).

### Mannanase

Mannase (E.C. 3.2.1.78) enzymes hydrolyse internal β-1,4 bonds in mannose polymers. "Mannanase" may be an alkaline mannanase of Family 5 or 26. Mannanase enzymes are known to be derived from wild-type from Bacillus or Humicola, particularly *B. agaradhaerens, B. licheniformis, B. halodurans, B. clausii,* or *H. insolens.* Suitable mannanases are described in WO 99/064619.

Commercially available mannanase enzymes include, but are not limited to, Mannaway^{®} (Novozymes AIS).

### Pectate lyase

Pectate lyase (E.C. 4.2.2.2) enzymes catalyze eliminative cleavage of (1->4)-alpha-D-galacturonan to give oligosaccharides with 4-deoxy-alpha-D-galact-4-enuronosyl groups at their non-reducing ends.

Pectate lyase enzymes have been described in patents and published patent applications including, but not limited to: WO 2004/090099. Pectate lyases are known to be derived from Bacillus, particularly *B. licheniformis* or *B. agaradhaerens,* or a variant derived of any of these, e.g. as described in US 6,124,127, WO 99/027083, WO 99/027084, WO 2002/006442, WO 02/092741, WO 03/095638.

Commercially available pectate lyase enzymes include: XpectTM, PectawashTM and PectawayTM (Novozymes A/S); PrimaGreenTM, EcoScour (DuPont).

### Nuclease

Nuclease (EC 3.1.21.1), also known as Deoxyribonuclease I, or Dnase, performs endonucleolytic cleavage to 5'-phosphodinucleotide and 5'-phosphooligonucleotide end-products.

Nuclease enzymes have been described in patents and published patent applications including, but not limited to: US 3,451,935, GB 1300596, DE 10304331, WO 2015/155350, WO 2015/155351, WO 2015/166075, WO 2015/181287, and WO 2015/181286.

Enzyme variants may be defined by their sequence identity when compared to a parent enzyme. Sequence identity usually is provided as "% sequence identity" or "% identity". To determine the percent-identity between two amino acid sequences in a first step a pairwise sequence alignment is generated between those two sequences, wherein the two sequences are aligned over their complete length (i.e., a pairwise global alignment). The alignment is generated with a program implementing the Needleman and Wunsch algorithm (J. Mol. Biol. (1979) 48, p. 443-453), preferably by using the program "NEEDLE" (The European Molecular Biology Open Software Suite (EMBOSS)) with the programs default parameters (gapopen=10.0, gapextend=0.5 and matrix=EBLOSUM62). The preferred alignment for the purpose of this invention is that alignment, from which the highest sequence identity can be determined.

After aligning the two sequences, in a second step, an identity value shall be determined from the alignment. Therefore, according to the present invention the following calculation of percent-identity applies:
%-identity = (identical residues / length of the alignment region which is showing the respective sequence of this invention over its complete length) *100. Thus sequence identity in relation to comparison of two amino acid sequences according to this embodiment is calculated by dividing the number of identical residues by the length of the alignment region which is showing the respective sequence of this invention over its complete length. This value is multiplied with 100 to give "%-identity".

For calculating the percent identity of two DNA sequences the same applies as for the calculation of percent identity of two amino acid sequences with some specifications. For DNA sequences encoding for a protein the pairwise alignment shall be made over the complete length of the coding region from start to stop codon excluding introns. For non-protein-coding DNA sequences the pairwise alignment shall be made over the complete length of the sequence of this invention, so the complete sequence of this invention is compared to another sequence, or regions out of another sequence. Moreover, the preferred alignment program implementing the Needleman and Wunsch algorithm (J. Mol. Biol. (1979) 48, p. 443-453) is " NEEDLE" (The European Molecular Biology Open Software Suite (EMBOSS)) with the programs default parameters (gapopen=10.0, gapextend=0.5 and matrix=EDNAFULL).

### Hormones

In another embodiment, the protein of interest may be a hormone such as, but not limited to, insulin, human growth hormone, somatostatin and insulin-like growth factor.

### Downstream processing

After the process of the present invention has been performed, the protein of interest may or may not be further purified from the fermentation broth. Thus, in one embodiment, the present invention refers to a fermentation broth comprising a protein of interest obtained by a fermentation process according to the present invention.

After the fermentation broth has been harvested, the desired product may be recovered and further purified by methods known in the art.

The desired protein of interest, preferably the desired protein, may be secreted (into the liquid fraction of the fermentation broth) or may not be secreted from the Bacillus cells (and therefore is comprised in the cells of the fermentation broth). Depending on this, the desired protein may be recovered from the liquid fraction of the fermentation broth or from cell lysates. Recovery of the desired protein can be achieved by methods known to those skilled in the art. Suitable methods for recovery of proteins from fermentation broth include but are not limited to collection, centrifugation, filtration, extraction, and precipitation. If the protein of interest precipitates or crystallizes in the fermentation broth or binds at least in part to the particulate matter of the fermentation broth additional treatment steps might be needed to release the protein of interest from the biomass or to solubilize protein of interest crystals and precipitates. US 6,316,240 B1 and WO 2008/110498 A1 describe a method for recovering a protein of interest, which precipitates and/or crystallizes during fermentation, from the fermentation broth. In case the desired protein is comprised in the cells of the fermentation broth release of the protein of interest from the cells might be needed. Release from the cells can be achieved for instance, but not being limited thereto, by cell lysis with techniques well known to the skilled person, e.g., lysozyme treatment, ultrasonic treatment, French press or combinations thereof.

The protein of interest, preferably the protein of interest, may be purified from the fermentation broth by methods known in the art. For example, a protein of interest may be isolated from the fermentation broth by conventional procedures including, but not limited to, centrifugation, filtration, extraction, spray-drying, evaporation, or precipitation. The isolated polypeptide may then be further purified by a variety of procedures known in the art including, but not limited to, chromatography (e.g., ion exchange, affinity, hydrophobic, chromatofocusing, and size exclusion), electrophoretic procedures (e.g., preparative isoelectric focusing (IEF), differential solubility (e.g., ammonium sulfate precipitation), or extraction (see, e.g., Protein Purification, J.-C. Janson and Lars Ryden, editors, VCH Publishers, New York, 1989). The purified polypeptide may then be concentrated by procedures known in the art including, but not limited to, ultrafiltration and evaporation, in particular, thin film evaporation.

The purified protein solution may be further processed to form a "protein formulation". "Protein formulation" means any non-complex formulation comprising a small number of ingredients, wherein the ingredients serve the purpose of stabilizing the proteins comprised in the protein formulation and/or the stabilization of the protein formulation itself. The term "protein stability" relates to the retention of protein activity as a function of time during storage or operation. The term "protein formulation stability" relates to the maintenance of physical appearance of the protein formulation during storage or operation as well as the avoidance of microbial contamination during storage or operation.

The protein formulation can be either solid or liquid. Protein formulations can be obtained by using techniques known in the art. For instance, without being limited thereto, solid enzyme formulations can be obtained by extrusion or granulation. Suitable extrusion and granulation techniques are known in the art and are described for instance in WO 94/19444 A1 and WO 97/43482 A1. "Liquid" in the context of enzyme formulation is related to the physical appearance at 20°C and 101.3 kPa. Liquid protein formulations may comprise amounts of enzyme in the range of 0.1% to 40% by weight, or 0.5% to 30% by weight, or 1% to 25% by weight, or 3% to 10%, all relative to the total weight of the enzyme formulation.

The protein as produced by the method of the present invention may be used in food, for example the protein can be an additive for baking. The protein can be used in feed, for example the protein is an animal feed additive. The protein can be used in the starch processing industry, for example amylases are used in the conversion of starch to ethanol or sugars (high fructose corn syrup) and other byproducts such as oil, dry distiller's grains, etc. The protein maybe used in pulp and paper processing, for example, the protein can be used for improving paper strength. In one embodiment, the protein produced by the methods of the present invention are used in detergent formulations or cleaning formulations. "Detergent formulation" or "cleaning formulation" means compositions designated for cleaning soiled material. Cleaning includes laundering and hard surface cleaning. Soiled material according to the invention includes textiles and/or hard surfaces.

### Specific embodiments

The following shows a list of specific embodiments of the invention:
In one embodiment, the invention relates to a process for culturing *Bacillus* cells producing a protein of interest, comprising the steps of:
(a) providing a pre-culture of said *Bacillus* cells;
(b) inoculating a production bioreactor with the pre-culture of step (a); and
(c) culturing the *Bacillus* cells in the production bioreactor under conditions conducive for the production of the protein of interest,
wherein the pre-culture of step (a) is performed in fed-batch mode, and
wherein the Bacillus cells are cells of Bacillus subtilis, Bacillus pumilus, Bacillus licheniformis, or Bacillus lentus.

In one embodiment, the invention relates to a process for culturing *Bacillus* cells producing a protein of interest, comprising the steps of:
(a) providing a pre-culture of said *Bacillus* cells;
(b) inoculating a production bioreactor with the pre-culture of step (a); and
(c) culturing the *Bacillus* cells in the production bioreactor under conditions conducive for the production of the protein of interest,

wherein the pre-culture of step (a) is performed in fed-batch mode; and
   wherein more than 200 g of a carbon source (preferably, a chemically defined carbon source) per liter of initial fermentation medium are added to the production bioreactor during culturing of the Bacillus cells in the production bioreactor, and
wherein the Bacillus cells are cells of Bacillus subtilis, Bacillus pumilus, Bacillus licheniformis, or Bacillus lentus.

In one embodiment, the invention relates to a process for culturing *Bacillus* cells producing a enyzme, comprising the steps of:
(a) providing a pre-culture of said *Bacillus* cells;
(b) inoculating a production bioreactor with the pre-culture of step (a); and
(c) culturing the *Bacillus* cells in the production bioreactor under conditions conducive for the production of the enzyme,
wherein the pre-culture of step (a) is performed in fed-batch mode, and
wherein the Bacillus cells are cells of Bacillus subtilis, Bacillus pumilus, Bacillus licheniformis, or Bacillus lentus.

In one embodiment, the invention relates to a process for culturing *Bacillus* cells producing a protease, comprising the steps of:
(a) providing a pre-culture of said *Bacillus* cells;
(b) inoculating a production bioreactor with the pre-culture of step (a); and
(c) culturing the *Bacillus* cells in the production bioreactor under conditions conducive for the production of the protease,
wherein the pre-culture of step (a) is performed in fed-batch mode, and
wherein the Bacillus cells are cells of Bacillus subtilis, Bacillus pumilus, Bacillus licheniformis, or Bacillus lentus.

In one embodiment, the invention relates to a process for culturing *Bacillus* cells producing an amylase, comprising the steps of:
(a) providing a pre-culture of said *Bacillus* cells;
(b) inoculating a production bioreactor with the pre-culture of step (a); and
(c) culturing the *Bacillus* cells in the production bioreactor under conditions conducive for the production of the amylase,
wherein the pre-culture of step (a) is performed in fed-batch mode, and
wherein the Bacillus cells are cells of Bacillus subtilis, Bacillus pumilus, Bacillus licheniformis, or Bacillus lentus.

In one embodiment, the invention relates to a process for culturing *Bacillus licheniformis* cells producing a protein of interest, comprising the steps of:
(a) providing a pre-culture of said *Bacillus licheniformis* cells;
(b) inoculating a production bioreactor with the pre-culture of step (a); and
(c) culturing the *Bacillus licheniformis* cells in the production bioreactor under conditions conducive for the production of the protein of interest,
wherein the pre-culture of step (a) is performed in fed-batch mode.

In one embodiment, the invention relates to a process for culturing *Bacillus licheniformis* cells producing a protease, comprising the steps of:
(a) providing a pre-culture of said *Bacillus licheniformis* cells;
(b) inoculating a production bioreactor with the pre-culture of step (a); and
(c) culturing the *Bacillus licheniformis* cells in the production bioreactor under conditions conducive for the production of the protease,
wherein the pre-culture of step (a) is performed in fed-batch mode.

In one embodiment, the invention relates to a process for culturing *Bacillus licheniformis* cells producing an amylase, comprising the steps of:
(a) providing a pre-culture of said *Bacillus licheniformis* cells;
(b) inoculating a production bioreactor with the pre-culture of step (a); and
(c) culturing the *Bacillus licheniformis* cells in the production bioreactor under conditions conducive for the production of the amylase,
wherein the pre-culture of step (a) is performed in fed-batch mode.

The invention is further illustrated in the following examples which are not intended to be in any way limiting to the scope of the invention as claimed. The numerous possible variations that are obvious to a person skilled in the art also fall within the scope of the invention.

### Examples

Unless otherwise stated the following experiments have been performed by applying standard equipment, methods, chemicals, and biochemicals as used in genetic engineering and fermentative production of chemical compounds by cultivation of microorganisms. See also Sambrook et al. (Molecular Cloning: A Laboratory Manual. 2nd edition, Cold Spring Harbor Laboratory, Cold Spring Harbor Laboratory Press, Cold Spring Harbor, NY, 1989) and Chmiel et al. (Bioprozesstechnik 1. Einführung in die Bioverfahrenstechnik, Gustav Fischer Verlag, Stuttgart, 1991).

For the first pre-culture described in the examples below (Examples 1-3), shake flasks containing a complex medium described in Table 1 was used. Shake flasks were inoculated from cryo stocks of *Bacillus licheniformis* comprising a gene coding for a protease or amylase and cultivated for 16h at 30°C at 200 rpm.

**Table 1:**

| *Compound* | *Formula* | *Concentration [g*/*L]* |
|---|---|---|
| Yeast extract | - | 7.4 |
| Sodium chloride | NaCl | 5.6 |
| Sucrose | C₁₂H₂₂O₁₁ | 45 |
| Potassium dihydrogen phosphate | KH₂PO₄ | 13.6 |
| Tubermin | - | 25 |

Before autoclaving, pH was set to 7.5 using NaOH.

For further pre-cultures and the main cultures in the production bioreactors described in the examples below, the *Bacillus licheniformis* cells from the first pre-culture were cultivated in a fermentation process using a chemically defined fermentation medium providing the components listed in Table 2 and Table 3.

**Table 2: Macroelements provided in the fermentation process**

| *Compound* | *Formula* | *Concentration [g*/*L initial volume]* |
|---|---|---|
| Citric acid | C₆H₈O₇ | 3.0 |
| Calcium sulfate | CaSO₄ | 0.7 |
| Monopotassium phosphate | KH₂PO₄ | 25 |
| Magnesium sulfate | MgSO₄*7H2O | 4.8 |
| Sodium hydroxide | NaOH | 4.0 |
| Ammonia | NH₃ | 1.3 |

**Table 3: Trace elements provided in the fermentation process**

| *Trace element* | *Symbol* | *Concentration [mM]* |
|---|---|---|
| Manganese | Mn | 24 |
| Zinc | Zn | 17 |
| Copper | Cu | 32 |
| Cobalt | Co | 1 |
| Nickel | Ni | 2 |
| Molybdenum | Mo | 0.2 |
| Iron | Fe | 38 |

The fermentation was started with a medium containing 8 g/l glucose. A solution containing 50% glucose was used as feed solution. The pH was adjusted during fermentation using ammonia.

### Example 1

*Bacillus* cells were used for the production of either a protease or an amylase. The seed train of the respective pre-cultures consisted of three seed fermenters. The first pre-culture in a shake flask was run with a complex medium as described above. The 2^{nd} pre-culture in a seed fermenter was inoculated with 10% of its volume from the first pre-culture and cultured in the chemically defined medium described above. The 2^{nd} pre-culture was run in batch mode for 16h or until glucose depletion which was indicated by a sharp increase in the dissolved oxygen signal and a rise in pH. The 3^{rd} pre-culture containing the chemically defined medium described above was inoculated with 10% of its volume from the 2^{nd} pre-culture. The 3^{rd} pre-culture was run in fed-batch mode using the chemically defined medium described above and a feed solution containing 50% glucose. The feed was started upon depletion of the initial amount of 8 g/l glucose which was indicated by an increase of culture pH by 0.2 pH units. The feed was added for 22h.

As a control, a pre-culture was conducted using the same number of seed fermenters and the same media, wherein the pre-culture was run completely in batch mode using a chemically defined medium containing 20 g/L glucose in the 2^{nd} and 3^{rd} pre-culture.

The volume of the inoculum of both the control pre-culture and the fed-batch pre-culture transferred to the production bioreactor was 10% of the volume of the fermentation medium initially present in the production bioreactor, respectively. Cultivation of the microbial cells in the production bioreactor was conducted in fed-batch mode using a chemically defined medium as described above. The feed with a feed solution containing 50% glucose was started upon depletion of an initial amount of 8 g/l glucose indicated by an increase of culture pH by 0.2 pH units and added until a product concentration of 10 g/kg (corresponds to 10 g/Liter) fermentation broth was reached.

Time until harvest was monitored using enzyme assays for protease and amylase on continuous samples. Protease Activity was determined using Succinyl - Ala - Ala - Pro - Phe - p-Nitroanilide (Suc-AAPF-pNA, short: AAPF) as substrate. pNA is cleaved from the substrate molecule at 30°C, pH 8.6 TRIS buffer. The rate of cleavage can be determined by the increase of the yellow color of free pNA in the solution by measuring OD₄₀₅, the optical density at 405 nm. Alpha-amylase activity was determined by a method employing the substrate Ethyliden-4-nitrophenyl-α-D-maltoheptaoside (EPS). D-maltoheptaoside is a blocked oligosaccharide which can be cleaved by an endo-amylase. Following the cleavage an alpha-glucosidase liberates a PNP molecule which has a yellow color and thus can be measured by visible spectophotometry at 405nm. Kits containing EPS substrate and alpha-glucosidase are available from Roche Costum Biotech (cat. No. 10880078t3) and are described in Lorentz K. et al. (2000), Clin. Chem., 46/5: 644 - 649. The slope of the time dependent absorption-curve is directly proportional to the specific activity (activity per mg enzyme) of the alpha-amylase in question under the given set of conditions.

The time point of harvest was defined as the time point when 10 g of protease or amylase/ kg fermentation broth was produced. The time until harvest was normalized to the control culture, i.e. to the time needed if a batch pre-culture was used before inoculation of the production bioreactor.

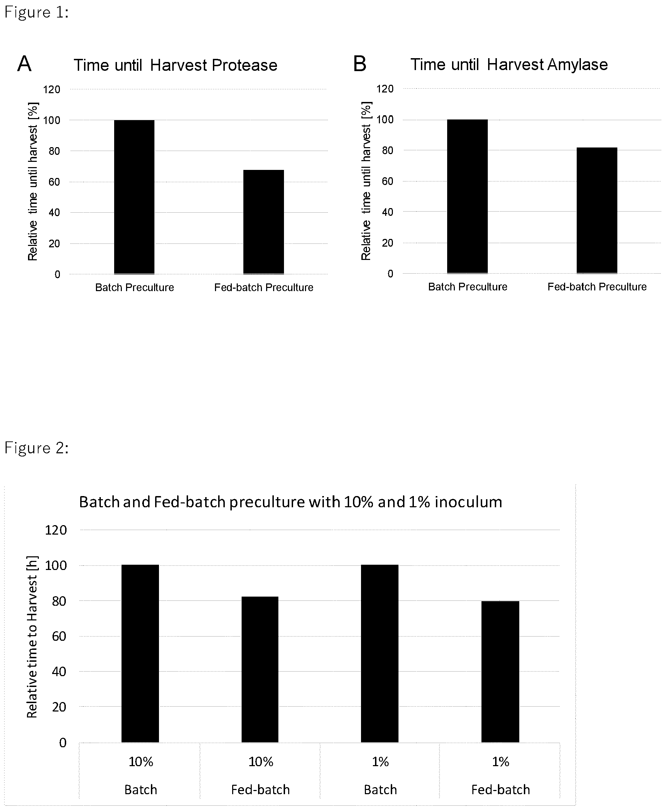

For the production of the protease, the time until harvest was reduced by about 30% when the pre-culture preceding the bioreactor was run in fed-batch mode compared to the pre-culture run completely in batch mode (see Figure 1A). For the production of the amylase, the time until harvest was reduced by around 20% when the pre-culture preceding the bioreactor was run in fed-batch mode compared to the pre-culture run completely in batch mode (see Figure 1B).

### Example 2

*Bacillus licheniformis* cells were used for the production of a protease. The seed train was performed as described in Example 1 for fed-batch pre-culture and batch pre-culture. The volume of the respective inoculum from the pre-culture for the production bioreactor was either 10% of the volume of the fermentation medium initially present in the production bioreactor or 1% of the volume of the fermentation medium initially present in the production bioreactor (Figure 2). Cultivation of the microbial cells in the production bioreactor was conducted in fed-batch mode. Time until harvest was monitored using an enzyme assay for protease on continuous samples as described in Example 1. The time of harvest, and thus the end of the fermentation process, was defined as the time point when 10 g of protease/ kg fermentation broth has been produced. The time until harvest was normalized to the control culture, i.e. to the time needed if a batch pre-culture is used.

Figure 2 shows that when the volume of the inoculum was 1% or 10%, the time until harvest was reduced by about 20% when the pre-culture was run in fed-batch mode.

### Example 3

*Bacillus licheniformis* cells were used for the production of a protease. In this Example, different media were tested in the final seed fermenter preceding the production bioreactor. Thus, the medium in the final seed fermenter of the seed-train was either a complex medium or a chemically defined medium. The pre-culture was then transferred to the production bioreactor. The volume of the inoculum was 10% of the volume of the fermentation medium initially present in the production bioreactor.

Cultivation of the microbial cells in the production bioreactor was conducted in fed-batch mode as described in Examples 1 and 2. Time until harvest was monitored using an enzyme assay for protease on continuous samples, using the assay described in Example 1. The time of harvest, and thus the end of the fermentation process, was defined as the time point when 10 g of product of interest/ kg fermentation broth has been produced. The time until harvest was normalized to the culture conducted with chemically defined media.

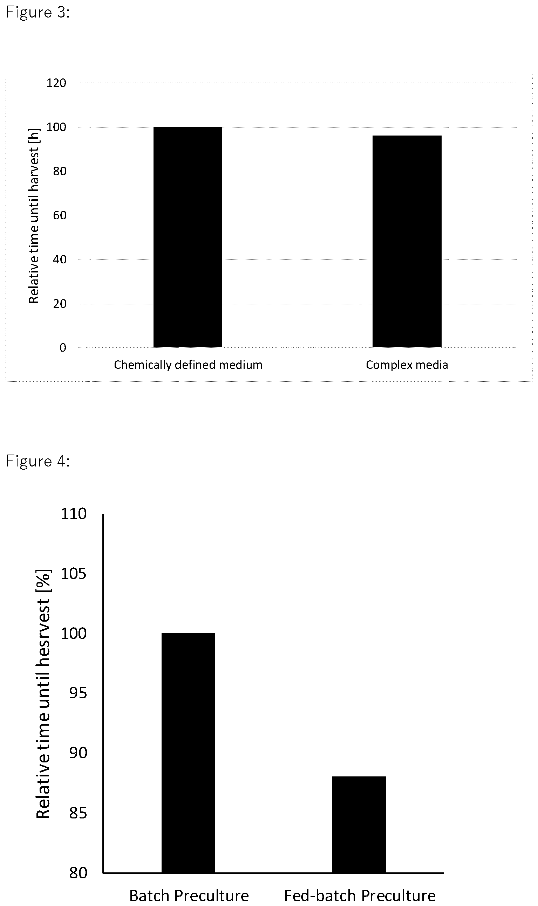

Figure 3 shows that the time until harvest was essentially the same when either a chemically defined medium or a complex medium was used in the fed-batch pre-culture.

### Example 4

*Bacillus subtilis* cells were used for the production of a protease. The seed train was performed as described in Example 1 for fed-batch pre-culture and batch pre-culture. The volume of the respective inoculum from the pre-culture for the production bioreactor was 10% of the volume of the fermentation medium initially present in the production bioreactor. Cultivation of the microbial cells in the production bioreactor was conducted in fed-batch mode. Time until harvest was monitored using an enzyme assay for protease on continuous samples as described in Example 1. The time of harvest, and thus the end of the fermentation process, was defined as the time point when 10 g of protease/ kg fermentation broth has been produced. The time until harvest was normalized to the control culture, i.e. to the time needed if a batch pre-culture is used.

The time until harvest was reduced by about 12% when the pre-culture preceding the bioreactor was run in fed-batch mode compared to the pre-culture run completely in batch mode (see Figure 4).

## Claims

1. A process for culturing Bacillus cells producing a protein of interest, comprising the steps of:
(a) providing a pre-culture of said Bacillus cells;
(b) inoculating a production bioreactor with the pre-culture of step (a); and
(c) culturing the Bacillus cells in the production bioreactor under conditions conducive for the production of the protein of interest,
wherein the pre-culture of step (a) is performed in fed-batch mode, and
wherein the Bacillus cells are cells of Bacillus subtilis, Bacillus pumilus, Bacillus licheniformis, or Bacillus lentus.

2. The process of claim 1, wherein the volume of the pre-culture used for inoculating the production bioreactor is not more than 15% of the volume of the fermentation medium present in the bioreactor.

3. The process of claim 1 and 2, wherein the pre-culture comprises at least two stages, all performed in fed-batch mode.

4. The process of any one of the preceding claims, wherein step c) is performed in batch mode, fed-batch mode or continuous fermentation mode.

5. The process of claim 4, wherein the Bacillus cells are cells of Bacillus licheniformis.

6. The process of any one of the preceding claims, wherein the protein of interest is an enzyme.

7. The fermentation process of claim 6, wherein the enzyme is selected from the list consisting of hydrolases, oxidases, isomerases, amylase, alpha-amylase, glucoamylase, pullulanase, protease, metalloprotease, peptidase, lipase, cutinase, acyl transferase, cellulase, endoglucanase, glucosidase, cellubiohydrolase, xylanase, xyloglucantransferase, xylosidase, mannanase, phytase, phosphatase, xylose isomerase, glucose isomerase, lactase, acetolactate decarboxylase, pectinase, pectin methylesterase, polygalacturonidase, lyase, pectate lyase, arabinase, arabinofuranosidase, galactanase, laccase, peroxidase, and asparaginase.

8. The process of any one of the preceding claims, wherein the protein of interest is secreted by the Bacillus cells into the fermentation broth.

9. The process of any one of the preceding claims, further comprising a step (d) of harvesting the protein when the concentration of the protein of interest is at least 10 g protein/kg fermentation broth.

10. A process for producing a protein of interest, comprising the steps of:
(a) providing a pre-culture of Bacillus cells producing said protein of interest;
(b) inoculating a production bioreactor with the pre-culture of step (a); and
(c) culturing the Bacillus cells in the production bioreactor under conditions conducive for the production of the protein of interest,
wherein the pre-culture of step (a) is performed in fed-batch mode, and
wherein the Bacillus cells are cells of Bacillus subtilis, Bacillus pumilus, Bacillus licheniformis, or Bacillus lentus.

11. A process for reducing the time until harvest in the fermentative production of a protein of interest, comprising the steps of:
(a) providing a pre-culture of Bacillus cells producing said protein of interest;
(b) inoculating a production bioreactor with the pre-culture of step (a); and
(c) culturing the Bacillus cells in the production bioreactor under conditions conducive for the production of the protein of interest,
wherein the pre-culture of step (a) is performed in fed-batch mode, and
wherein the Bacillus cells are cells of Bacillus subtilis, Bacillus pumilus, Bacillus licheniformis, or Bacillus lentus.

## Patentansprüche

1. Verfahren zur Kultivierung von Bacillus-Zellen, die ein Protein von Interesse produzieren, umfassend die Schritte:
(a) Bereitstellen einer Vorkultur der Bacillus-Zellen;
(b) Animpfen eines Produktionsbioreaktors mit der Vorkultur von Schritt (a); und
(c) Kultivieren der Bacillus-Zellen im Produktionsbioreaktor unter Bedingungen, die der Produktion des Proteins von Interesse förderlich sind,
wobei die Vorkultur von Schritt (a) im Fed-batch-Modus erfolgt und wobei es sich bei den Bacillus-Zellen um Zellen von Bacillus subtilis, Bacillus pumilus, Bacillus licheniformis oder Bacillus lentus handelt.

2. Verfahren nach Anspruch 1, wobei das Volumen der zum Animpfen des Produktionsbioreaktors verwendeten Vorkultur nicht mehr als 15% des im Bioreaktor vorliegenden Fermentationsmediums beträgt.

3. Verfahren nach Anspruch 1 und 2, wobei die Vorkultur wenigstens zwei Stufen umfasst, die jeweils im Fed-batch-Modus erfolgen.

4. Verfahren nach einem der vorhergehenden Ansprüche, wobei Schritt c) im Batch-Modus, Fed-batch-Modus oder Kontinuierliche-Fermentation-Modus erfolgt.

5. Verfahren nach Anspruch 4, wobei es sich bei den Bacillus-Zellen um Zellen von Bacillus licheniformis handelt.

6. Verfahren nach einem der vorhergehenden Ansprüche, wobei es sich bei dem Protein von Interesse um ein Enzym handelt.

7. Fermentationsverfahren nach Anspruch 6, wobei das Enzym aus der aus Hydrolasen, Oxidasen, Isomerasen, Amylase, Alpha-Amylase, Glucoamylase, Pullulanase, Protease, Metalloprotease, Peptidase, Lipase, Cutinase, Acyltransferase, Cellulase, Endoglucanase, Glucosidase, Cellubiohydrolase, Xylanase, Xyloglucantransferase, Xylosidase, Mannanase, Phytase, Phosphatase, Xylose-Isomerase, Glucose-Isomerase, Lactase, Acetolactat-Decarboxylase, Pektinase, Pektin-Methylesterase, Polygalacturonidase, Lyase, Pektatlyase, Arabinase, Arabinofuranosidase, Galactanase, Laccase, Peroxidase und Asparaginase bestehenden Liste ausgewählt ist.

8. Verfahren nach einem der vorhergehenden Ansprüche, wobei das Protein von Interesse von den Bacillus-Zellen in die Fermentationsbrühe sezerniert wird.

9. Verfahren nach einem der vorhergehenden Ansprüche, ferner umfassend einen Schritt (d), bei dem das Protein geerntet wird, wenn die Konzentration des Proteins von Interesse wenigstens 10 g Protein pro kg Fermentationsbrühe beträgt.

10. Verfahren zur Produktion eines Proteins von Interesse, umfassend die Schritte:
(a) Bereitstellen einer Vorkultur von Bacillus-Zellen, die das Protein von Interesse produzieren;
(b) Animpfen eines Produktionsbioreaktors mit der Vorkultur von Schritt (a); und
(c) Kultivieren der Bacillus-Zellen im Produktionsbioreaktor unter Bedingungen, die der Produktion des Proteins von Interesse förderlich sind,
wobei die Vorkultur von Schritt (a) im Fed-batch-Modus erfolgt und wobei es sich bei den Bacillus-Zellen um Zellen von Bacillus subtilis, Bacillus pumilus, Bacillus licheniformis oder Bacillus lentus handelt.

11. Verfahren zur Verkürzung der Zeit bis zur Ernte bei der fermentativen Produktion eines Proteins von Interesse, umfassend die Schritte:
(a) Bereitstellen einer Vorkultur von Bacillus-Zellen, die das Protein von Interesse produzieren;
(b) Animpfen eines Produktionsbioreaktors mit der Vorkultur von Schritt (a); und
(c) Kultivieren der Bacillus-Zellen im Produktionsbioreaktor unter Bedingungen, die der Produktion des Proteins von Interesse förderlich sind,
wobei die Vorkultur von Schritt (a) im Fed-batch-Modus erfolgt und wobei es sich bei den Bacillus-Zellen um Zellen von Bacillus subtilis, Bacillus pumilus, Bacillus licheniformis oder Bacillus lentus handelt.

## Revendications

1. Procédé pour la culture de cellules de Bacillus produisant une protéine d'intérêt, comprenant les étapes de :
(a) fourniture d'une pré-culture desdites cellules de Bacillus ;
(b) inoculation d'un bioréacteur de production avec la pré-culture de l'étape (a) ; et
(c) culture des cellules de Bacillus dans le bioréacteur de production dans des conditions propices à la production de la protéine d'intérêt,
la pré-culture de l'étape (a) étant réalisée en mode d'alimentation par lots, et
les cellules de Bacillus étant des cellules de Bacillus subtilis, Bacillus pumilus, Bacillus licheniformis, ou Bacillus lentus.

2. Procédé selon la revendication 1, le volume de la pré-culture utilisée pour l'inoculation du bioréacteur de production n'étant pas supérieur à 15 % du volume du milieu de fermentation présent dans le bioréacteur.

3. Procédé selon la revendication 1 et 2, la pré-culture comprenant au moins deux stades, tous réalisés en mode d'alimentation par lots.

4. Procédé selon l'une quelconque des revendications précédentes, l'étape c) étant réalisée en mode par lots, en mode d'alimentation par lots ou en mode de fermentation continue.

5. Procédé selon la revendication 4, les cellules de Bacillus étant des cellules de Bacillus licheniformis.

6. Procédé selon l'une quelconque des revendications précédentes, la protéine d'intérêt étant une enzyme.

7. Procédé de fermentation selon la revendication 6, l'enzyme étant choisie dans la liste constituée par des hydrolases, des oxydases, des isomérases, une amylase, une alpha-amylase, une glucoamylase, une pullulanase, une protéase, une métalloprotéase, une peptidase, une lipase, une cutinase, une acyltransférase, une cellulase, une endoglucanase, une glucosidase, une cellubiohydrolase, une xylanase, une xyloglucanetransférase, une xylosidase, une mannanase, une phytase, une phosphatase, une xylose isomérase, une glucose isomérase, une lactase, une acétolactate décarboxylase, une pectinase, une pectine méthylestérase, une polygalacturonidase, une lyase, une pectate lyase, une arabinase, une arabinofuranosidase, une galactanase, une laccase, une peroxydase, et une asparaginase.

8. Procédé selon l'une quelconque des revendications précédentes, la protéine d'intérêt étant sécrétée par les cellules de Bacillus dans le bouillon de fermentation.

9. Procédé selon l'une quelconque des revendications précédentes, comprenant en outre une étape (d) de récolte de la protéine lorsque la concentration de la protéine d'intérêt est d'au moins 10 g de protéine/kg de bouillon de fermentation.

10. Procédé pour la production d'une protéine d'intérêt, comprenant les étapes de :
(a) fourniture d'une pré-culture de cellules de Bacillus produisant ladite protéine d'intérêt ;
(b) inoculation d'un bioréacteur de production avec la pré-culture de l'étape (a) ; et
(c) culture des cellules de Bacillus dans le bioréacteur de production dans des conditions propices à la production de la protéine d'intérêt,
la pré-culture de l'étape (a) étant réalisée en mode d'alimentation par lots, et
les cellules de Bacillus étant des cellules de Bacillus subtilis, Bacillus pumilus, Bacillus licheniformis, ou Bacillus lentus.

11. Procédé pour la réduction du temps jusqu'à la récolte dans la production, par fermentation, d'une protéine d'intérêt, comprenant les étapes de :
(a) fourniture d'une pré-culture de cellules de Bacillus produisant ladite protéine d'intérêt ;
(b) inoculation d'un bioréacteur de production avec la pré-culture de l'étape (a) ; et
(c) culture des cellules de Bacillus dans le bioréacteur de production dans des conditions propices à la production de la protéine d'intérêt,
la pré-culture de l'étape (a) étant réalisée en mode d'alimentation par lots, et
les cellules de Bacillus étant des cellules de Bacillus subtilis, Bacillus pumilus, Bacillus licheniformis, ou Bacillus lentus.
